(19) Europäisches Patentamt
European Patent Office
Office européen des brevets

(11) EP 2 066 835 B1

(12) **EUROPEAN PATENT SPECIFICATION**

(45) Date of publication and mention of the grant of the patent:
**21.11.2012 Bulletin 2012/47**

(21) Application number: **07809760.7**

(22) Date of filing: **21.06.2007**

(51) Int Cl.:
***D04H 13/00*** *(2006.01)* ***A47L 13/16*** *(2006.01)*
***A47L 13/17*** *(2006.01)* ***A61K 8/02*** *(2006.01)*
***B32B 5/26*** *(2006.01)* ***D21H 21/14*** *(2006.01)*
***D21H 25/00*** *(2006.01)*

(86) International application number:
**PCT/US2007/014478**

(87) International publication number:
**WO 2008/156455 (24.12.2008 Gazette 2008/52)**

(54) **TREATED NONWOVEN WEBS CONTAINING SYNTHETIC AND PULP FIBERS**

BEHANDELTE VLIESSTOFFE MIT SYNTHESE- UND ZELLSTOFFFASERN

BANDES NON TISSÉES TRAITÉES CONTENANT DES FIBRES SYNTHÉTIQUES ET DES FIBRES DE PÂTE À PAPIER

(84) Designated Contracting States:
**ES FR GB IT**

(43) Date of publication of application:
**10.06.2009 Bulletin 2009/24**

(73) Proprietor: **KIMBERLY-CLARK WORLDWIDE, INC.**
**Neenah, WI 54956 (US)**

(72) Inventors:
- **DYER, Thomas, Joseph**
  **Neenah, Wisconsin 54956 (US)**
- **FETNER, Christopher, B.**
  **Neenah, Wisconsin 54956 (US)**
- **RUNGE, Troy, M.**
  **Neenah, Wisconsin 54956 (US)**
- **SCHMIDT, Michael, A.**
  **Alpharetta, Georgia 30022 (US)**
- **PATE, Courtney, E.**
  **Greenville, WI 54942 (US)**

(74) Representative: **Zimmermann & Partner**
**Postfach 330 920**
**80069 München (DE)**

(56) References cited:

| | |
|---|---|
| **WO-A-2005/021869** | **WO-A-2007/070145** |
| **WO-A1-2008/068653** | **WO-A2-2007/075356** |
| **WO-A2-2008/068659** | **US-A1- 2004 099 389** |
| **US-A1- 2004 118 544** | **US-A1- 2005 100 754** |
| **US-A1- 2005 136 766** | **US-A1- 2007 295 464** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 066 835 B1

## Description

## BACKGROUND

Saturated or pre-moistened wiping products have been used in a variety of wiping and polishing applications. Perhaps the most common form is a stack of individual, folded sheets packaged in a plastic container for use as baby wipes. Wet wipes are also available containing antimicrobial compositions for cleaning desired surfaces. Wet wipes are typically used only once and then discarded.

Wet wipes designed to clean or disinfect adjacent surfaces are typically made containing synthetic fibers and/or water insoluble adhesives or binders. For instance, many wet wipe materials are made from airlaid webs that have been treated with a water insoluble adhesive or spunlace webs containing water insoluble synthetic fibers. Wet wipe materials may also contain meltspun webs, such as meltblown webs, spunbond webs and laminates thereof.

For example, in the past, various wiping products have been made from coform webs. Coform webs are made from a combination of synthetic fibers and pulp fibers. For example, in one embodiment, coform webs are made by injecting natural fibers, such as pulp fibers, into a stream of meltblown fibers as the meltblown fibers are being formed. Coform processes, for instance, are disclosed in U.S. Patent No. 4,818,464 and in U.S. Patent No. 4,100,324.

Coform webs generally have good bulk and strength characteristics and are typically durable when wet. Coform webs, however, can have a tendency to produce lint and are generally perceived not to be as soft as other competing products.

In view of the above, a need currently exists for a coform web that has improved lint properties and/or has improved softness characteristics.

US 2004/0099389 A1 relates to a paper product comprising a softened, uncreped, through-dried paper web comprising papermaking fibers and up to 50% synthetic fibers; a bonding material printed on a first side of the paper web in a first pattern; and wherein the printed paper web has a bulk of greater than about 7 cc/g. Disclosed bonding materials include hot melts, polymers, resins, elastomeric and thermoplastic compositions.

US 2004/0118544 A1 relates to a process for forming a wiping product, wherein a base web having a consistency of at least 50% is conveyed from a first moving surface to a second moving surface moving slower than the first moving surface, causing the base web to shear and decrease in stiffness. The process may comprise the step of applying a bonding material to at least one side of the base web prior to conveying the base web from the first moving surface to the second moving surface. Suitable bonding materials include conventional creping adhesives, latex compositions, non-latex adhesives and hot melt adhesives.

WO 2007/0700145 A1, published June 21, 2007, relates to tissue products containing an additive composition.

WO 2007/075356 A2, published July 5, 2007, describes a coform material which may contain a cellulosic material in an amount from about 10% by weight to about 80% by weight. Furthermore, from WO 2007/075356 A2 a method for producing a tissue product is known, whereby an additive composition is applied to a first side of the tissue web. The additive composition comprises an olefin polymer, wherein the olefin polymer comprises an alpha-olefin interpolymer of ethylene and at least one comonomer selected from the group consisting of a $C_4$ to $C_{20}$ linear, branched or cyclic diene, vinyl acetate, and a compound represented by the formula $H_2C{=}CHR$, wherein R is a $C_1$ to $C_{20}$ linear, branched or cyclic alkyl group or a $C_6$ to $C_{20}$ aryl group, or the alpha-olefin polymer comprises a copolymer of propylene with at least one comonomer selected from the group consisting of ethylene, a $C_4$ to $C_{20}$ linear, branched or cyclic diene, and a compound represented by the formula $H_2C{=}CHR$, wherein R is a $C_1$ to $C_{20}$ linear, branched or cyclic alkyl group or a $C_6$ to $C_{20}$ aryl group and wherein the additive composition further comprises a dispersing agent, wherein the dispersing agent comprises a carboxylic acid, a salt of a carboxylic acid, a carboxylic acid ester, a salt of a carboxylic ester, or an ethylene-carboxylic acid copolymer.

Similarly, WO 2008/068653 A1, published June 12, 2008, discloses a coform material which may contain a cellulosic material in an amount from about 10% by weight to about 80% by weight. Moreover, WO 2008/068653 A1 describes a tissue product comprising an additive composition. The additive composition comprises a non-fibrous olefin polymer. The olefin polymer comprises an alpha-olefin interpolymer of ethylene and at least one comonomer selected from the group consisting of a $C_4$ to $C_{20}$ linear, branched or cyclic diene, vinyl acetate, and a compound represented by the formula $H_2C{=}CHR$, wherein R is a $C_1$ to $C_{20}$ linear, branched or cyclic alkyl group or a $C_6$ to $C_{20}$ aryl group, or the alpha-olefin polymer comprises a copolymer of propylene with at least one comonomer selected from the group consisting of ethylene, a $C_4$ to $C_{20}$ linear, branched or cyclic diene, and a compound represented by the formula $H_2C{=}CHR$, wherein R is a $C_1$ to $C_{20}$ linear, branched or cyclic alkyl group or a $C_6$ to $C_{20}$ aryl group.

Also WO 2008/068659 A2, published June 12, 2008, describes a coform material which may contain a cellulosic material in an amount from about 10% by weight to about 80% by weight. In addition, WO 2008/068659 A2 discloses that additive compositions were applied to samples. One of the additives used was AFFINITY™ EG8200 plastomer, which is an alpha-olefin interpolymer comprising an ethylene and octane copolymer that was obtained from The Dow Chemical Company of Midland, Michigan, U.S.A. A further additive used was PRIMACOR™5980i copolymer, which is

an ethylene-acrylic acid copolymer also obtained from The Dow Chemical Company. The ethylene-acrylic acid copolymer can serve not only as a thermoplastic polymer but also as a dispersing agent. A further dispersing agent used was INDUSTRENE® 106, which comprises oleic acid, which is marketed by Chemtura Corporation, Middlebury, Connecticut. PRIMACOR™ 5980i copolymer contains 20.5% by weight acrylic acid and has a melt flow rate of 13.75 g/10 min at 125°C and 2.16 kg as measured by ASTM D1238. AFFINITY™ EG8200G plastomer has a density of 0.87 g/cc as measured by ASTM D792 and has a melt flow rate of 5 g/10 min at 190°C and 2.16 kg as measured by ASTM D1238.

Moreover, from WO 2008/068652 A2, published June 12, 2008, a coform material is known which may contain cellulosic material in an amount from about 10% by weight to about 80% by weight. Furthermore, WO 2008/068652 discloses an additive composition which comprises a non-fibrous olefin polymer. The olefin polymer comprises an alpha-olefin interpolymer of ethylene and at least one comonomer selected from the group consisting of a $C_4$ to $C_{20}$ linear, branched or cyclic diene, vinyl acetate, and a compound represented by the formula $H_2C{=}CHR$, wherein R is a $C_1$ to $C_{20}$ linear, branched or cyclic alkyl group or a $C_6$ to $C_{20}$ aryl group, or the alpha-olefin polymer comprises a copolymer of propylene with at least one comonomer selected from the group consisting of ethylene, a $C_4$ to $C_{20}$ linear, branched or cyclic diene, and a compound represented by the formula $H_2C{=}CHR$, wherein R is a $C_1$ to $C_{20}$ linear, branched or cyclic alkyl group or a $C_6$ to $C_{20}$ aryl group.

## SUMMARY

In general, the present disclosure is directed to wet and dry wiping products having improved properties due to the presence of an additive composition. The wiping product comprises a coform web containing synthetic and pulp fibers. The product may contain one ply or may contain multiple plies. In accordance with the present disclosure, the additive composition can be incorporated into the sheet-like product in order to enhance various properties. For instance, incorporating the additive composition into the product can reduce lint and pilling while increasing softness. In particular, the additive composition can improve the hand feel of the product and make the product more drapable. In addition, the additive composition can also increase strength and enhance the ability of the product to capture dirt particles when wiped against an adjacent surface.

The additive composition may comprise, for instance, an aqueous dispersion containing a thermoplastic resin. The additive composition is applied topically to a base sheet.

The additive composition may comprise a non-fibrous olefin polymer. The additive composition, for instance, may comprise a film-forming composition and the olefin polymer may comprise an interpolymer of ethylene and at least one comonomer comprising an alkene, such as 1-octene. The additive composition also contains a dispersing agent, such as a carboxylic acid. Examples of particular dispersing agents, for instance, include fatty acids, such as oleic acid or stearic acid.

In one particular embodiment, the additive composition may contain an ethylene and octene copolymer in combination with an ethylene-acrylic acid copolymer. The ethylene-acrylic acid copolymer is not only a thermoplastic resin, but may also serve as a dispersing agent. The ethylene and octene copolymer may be present in combination with the ethylene-acrylic acid copolymer in a weight ratio of from about 1:10 to about 10:1, such as from about 2:3 to about 3:2.

The olefin polymer composition may exhibit a crystallinity of less than about 50%, such as less than about 20%. The olefin polymer may also have a melt index of less than about 1000 g/10 min, such as less than about 700 g/10 min. The olefin polymer may also have a relatively small particle size, such as from about 0.1 micrometre to about 5 micrometres when contained in an aqueous dispersion.

In an alternative embodiment, the additive composition may contain an ethylene-acrylic acid copolymer. The ethylene-acrylic acid copolymer is present in the additive composition in combination with a dispersing agent, such as a fatty acid.

In one embodiment, the additive composition can be topically applied to one or both sides of a base sheet. Once applied to a base sheet, it has been discovered that the additive composition may form a discontinuous but interconnected film depending upon the amount applied to the sheet. In this manner, the additive composition increases the strength of the sheet without significantly interfering with the ability of the sheet to absorb fluids. For example, the discontinuous film that is formed includes openings that allow liquids to be absorbed by the sheet.

In other embodiments, the additive composition may be applied to a web in relatively light amounts such that the additive composition forms discrete treated areas on the surface of the web. Even at such low amounts, however, the additive composition can still enhance one or more properties of the web.

In one embodiment, the additive composition does not substantially penetrate into the base sheet when applied. For instance, the additive composition penetrates the web in an amount less than about 30% of the thickness of the web, such as less than about 20%, such as less than about 10% of the thickness of the web. By remaining primarily on the surface of the web, the additive composition does not interfere with the liquid absorption capacity properties of the web. Further, the additive composition does not substantially increase the stiffness of the web.

In still another embodiment, the additive composition can be incorporated into the center of the web or throughout the thickness of the web.

The base sheet treated with the additive composition in accordance with the present disclosure comprises a coform web. The coform web contains synthetic fibers combined with pulp fibers. In addition to improving the above described properties of the coform sheet, incorporating the additive composition into the coform material may also provide various other benefits and advantages. For instance, the presence of the additive composition may allow for the production of coform webs having a relatively light basis weight while still retaining sufficient strength. The basis weight of the coform web, for instance, in one embodiment may be less than about 50 g/$m^2$ as from about 15 g/$m^2$ to about 40 g/$m^2$ , such as from about 20 g/$m^2$ to about 30 g/$m^2$

In addition, since the additive composition reduces the amount of lint given off by the coform sheet, coform materials may be made according to the present disclosure that have a relatively large amount of pulp fibers. For example, pulp fibers may be present in the coform material in an amount greater than 70% by weight, such as greater than about 75% by weight, such as greater than about 80% by weight, such as greater than about 85% by weight, such as even greater than about 90% by weight. For instance, the coform material may contain pulp fibers in an amount from about 70% to about 90% by weight.

The manner in which the additive composition is incorporated into the coform material can vary depending upon the particular application. For instance, the additive composition may be incorporated into the coform material during its formation. Alternatively, the additive composition can be applied topically to the coform material. When applied topically, the additive composition can be applied to one side of the coform material or to both sides of the coform material.

In one embodiment, the additive composition may be applied to one side of the sheet for adhering the sheet to a creping drum and for creping the sheet from the drum surface. In this embodiment, for instance, the additive composition may be applied to one side of the sheet according to a pattern. The pattern may comprise, for instance, a pattern of discrete shapes, a reticulated pattern, or a combination of both. In order to apply the additive composition to the sheet, the additive composition may be printed onto the sheet according to the pattern. For instance, in one embodiment, a rotogravure printer may be used.

In an alternative embodiment, the additive composition may be sprayed onto one side of the sheet or sprayed onto the creping drum for adhering the sheet to the drum. Once adhered to the drum, the sheet can then be creped from the drum. Creping the sheet can increase bulk and softness.

The additive composition may be applied to one side of the base sheet in an amount from about 0.1% to about 50% by weight such as from about 0.1 % to about 30% by weight. In some embodiments, after the additive composition is applied to the sheet, the sheet can be dried at a temperature in the range of equal to or greater than the melting point temperature of the base polymer in the additive composition.

When the base sheet is adhered to a creping drum, if desired, the creping drum may be heated. For instance, the creping surface may be heated to a temperature of from about 80°C to about 200°C, such as from about 100°C to about 150°C. The additive composition may be applied only to a single side of the sheet or may be applied to both sides of the sheet according to the same or different patterns. When applied to both sides of the sheet, both sides of the sheet may be creped from a creping drum or only one side of the sheet may be creped.

In one embodiment, treated coform materials made in accordance with the present disclosure may be incorporated into a laminate. For instance, the treated coform material can be laminated to a tissue web, such as a wetlaid web. The tissue web, for instance, may comprise any suitable tissue product made in any suitable manner. Laminating the coform material to a tissue web produces a product having two distinct wiping surfaces with different pore structures and topography.

Once applied to or incorporated into a base sheet, such as a coform web in accordance with the present disclosure, the base sheet may be embossed by being fed through a nip formed between two embossing rolls or between an embossing roll and a smooth roll. The embossing elements contact the web at a pressure and/or temperature sufficient to soften the thermoplastic polymer contained within the additive composition and cause the polymer to flow forming defined embossments.

In other embodiments, however, embossing the web may not be preferred. For instance, when the base sheet is creped, it may be desirable not to emboss the web so as to preserve the surface characteristics of the sheet material that are created during the creping process.

After the base sheet is formed, in one embodiment, the sheet may be premoistened with a wiping solution. In general, any suitable wiping solution may be used in accordance with the present disclosure. For instance, in one embodiment, the wiping solution may comprise water in combination with at least one surfactant and/or at least one emollient. In an alternative embodiment, the wiping solution may contain water in combination with an alcohol. Various other and sundry ingredients may be present in the wiping solution such as aloe, a preservative, a glycol, an antimicrobial agent, a fragrance, and the like.

Other features and aspects of the present invention are discussed in greater detail below.

## BRIEF DESCRIPTION OF THE DRAWINGS

A full and enabling disclosure of the present invention, including the best mode thereof to one of ordinary skill in the art, is set forth more particularly in the remainder of the specification, including reference to the accompanying figures in which:

Figure 1 is a schematic diagram of a tissue web forming machine, illustrating the formation of a stratified tissue web having multiple layers in accordance with the present disclosure;
Figure 2 is a schematic diagram of one embodiment of a process for applying additive compositions to each side of a tissue web and creping one side of the web in accordance with the present disclosure;
Figure 3 is a plan view of one embodiment of a pattern that is used to apply additive compositions to tissue webs made in accordance with the present disclosure;
Figure 4 is another embodiment of a pattern that is used to apply additive compositions to tissue webs in accordance with the present disclosure;
Figure 5 is a plan view of another alternative embodiment of a pattern that is used to apply additive compositions to tissue webs in accordance with the present disclosure;
Figure 6 is a schematic diagram of an alternative embodiment of a process for applying an additive composition to one side of the tissue web and creping one side of the web in accordance with the present disclosure;
Figure 7 is a schematic diagram of still another embodiment of a process for applying an additive composition to one side of a tissue web and creping one side of the web in accordance with the present disclosure;
Figure 8 is a schematic diagram of still another embodiment of a process for applying an additive composition to one side of a tissue web and creping one side of the web in accordance with the present disclosure;
Figure 9 is a diagram illustrating the equipment used to perform a Stick-Slip Test;
Figures 10 and 11 are the results obtained in Example Nos. 2 and 3 below.

Repeat use of reference characters in the present specification and drawings is intended to represent same or analogous features or elements of the present disclosure.

## DETAILED DESCRIPTION

It is to be understood by one of ordinary skill in the art that the present discussion is a description of exemplary embodiments only, and is not intended as limiting the broader aspects of the present disclosure.

In general, the present disclosure is directed to the incorporation of an additive composition into a base sheet, such as a wiping product, in order to enhance various properties of the base sheet. The base sheet comprises acoform web. The additive composition may comprise a polyolefin dispersion. For example, the polyolefin dispersion may contain polymeric particles having a relatively small size, such as less than about 5 micrometers, in an aqueous medium when applied or incorporated into a base sheet. Once dried, however, the polymeric particles are generally indistinguishable. For example, in one embodiment, the additive composition may comprise a film-forming composition that forms a discontinuous film and/or forms discrete treated areas on the base sheet. The polyolefin dispersion also contains a dispersing agent.

As will be described in greater detail below, the additive composition can be incorporated into a base sheet using various techniques and during different stages of production of the sheet. The additive composition can be combined with the fibers that are used to form the sheet. The additive composition is topically applied to a base sheet. For example, the additive composition may be applied to a sheet during a creping operation. In particular, the additive composition has been found well-suited for adhering a base sheet to a creping surface during a creping process.

The use of the additive composition containing a polyolefin dispersion has been found to provide various benefits and advantages depending upon the particular embodiment. For example, the additive composition enhances the ability of a base sheet to clean surfaces and capture dirt particles. In particular, dirt particles are attached and held to the base sheet by the additive composition. Although unknown, it is believed that the additive composition produces triboelectric properties that are well suited for attracting and holding dirt particles. In particular, it is believed that the additive composition creates a static charge and also increases the coefficient of friction which enhances the ability of a base sheet to clean an adjacent surface. As will also be described below, the additive composition increases the coefficient of friction but yet glides across a surface once motion has started.

Once present on the base sheet, the additive composition is capable of enhancing the cleaning performance of the base sheet on multiple surfaces such as glass surfaces, plastic surfaces, wood surfaces, metal surfaces, one's skin, and the like. Further, the additive composition does not create any streaking or leave any residue. When applied to a web containing pulp fibers, the additive composition also reduces the amount of lint produced by the product.

As described above the base sheet treated in accordance with the present disclosure comprises a coform web.

In the coform process, at least one meltblown diehead is arranged near a chute through which other materials are added to a meltblown web while it is forming. Such other materials may be natural fibers, superabsorbent particles, natural polymer fibers (for example, rayon) and/or synthetic polymer fibers (for example, polypropylene or polyester), for example, where the fibers may be of staple length.

Coform processes are shown in commonly assigned US Patents 4,818,464 to Lau and 4,100,324 to Anderson et al.-Webs produced by the coform process are generally referred to as coform materials. More particularly, one process for producing coform nonwoven webs involves extruding a molten polymeric material through a die head into fine streams and attenuating the streams by converging flows of high velocity heated gas (usually air) supplied from nozzles to break the polymer streams into discontinuous microfibers of small diameter. The die head, for instance, can include at least one straight row of extrusion apertures. In general, the microfibers may have an average fiber diameter of up to about 10 micrometers. Theaverage diameter of the microfibers can be generally greater than about 1 micrometer, such as from about 2 micrometers to about 5 micrometers. While the microfibers are predominantly discontinuous, they generally have a length exceeding that normally associated with staple fibers.

In order to combine the molten polymer fibers with another material, such as pulp fibers, a primary gas stream is merged with a secondary gas stream containing the individualized wood pulp fibers. Thus, the pulp fibers become integrated with the polymer fibers in a single step. The wood pulp fibers can have a length of from about 0.5 millimeters to about 10 millimeters. The integrated air stream is then directed onto a forming surface to air form the nonwoven fabric. The nonwoven fabric, if desired, may be passed into the nip of a pair of vacuum rolls in order to further integrate the two different materials.

Natural fibers that may be combined with the meltblown fibers include wool, cotton, flax, hemp and wood pulp. Wood pulps include standard softwood fluffing grade such as CR-1654 (US Alliance Pulp Mills, Coosa, Alabama). Pulp may be modified in order to enhance the inherent characteristics of the fibers and their processability. Curl may be imparted to the fibers by methods including chemical treatment or mechanical twisting. Curl is typically imparted before crosslinking or stiffening. Pulps may be stiffened by the use of crosslinking agents such as formaldehyde or its derivatives, glutaraldehyde; epichlorohydrin, methylolated compounds such as urea or urea derivatives, dialdehydes such as maleic anhydride, non-methylolated urea derivatives, citric acid or other polycarboxylic acids. Pulp may also be stiffened by the use of heat or caustic treatments such as mercerization. Examples of these types of fibers include NHB416 which is a chemically crosslinked southern softwood pulp fibers which enhances wet modulus, available from the Weyerhaeuser Corporation of Tacoma, WA. Other useful pulps are debonded pulp (NF405) and non-debonded pulp (NB416) also from Weyerhaeuser. HPZ3 from Buckeye Technologies, Inc of Memphis, TN, has a chemical treatment that sets in a curl and twist, in addition to imparting added dry and wet stiffness and resilience to the fiber. Another suitable pulp is Buckeye HP2 pulp and still another is IP Supersoft from International Paper Corporation. Suitable rayon fibers are 1.5 denier Merge 18453 fibers from Acordis Cellulose Fibers Incorporated of Axis, Alabama.

In addition to enhancing the cleaning ability of the web and to reducing lint, applying the additive composition to a coform material can improve various other properties. For example, the additive composition can increase the strength of the product without significantly adversely impacting the stiffness of the material or other properties of the material. In fact, incorporating the additive composition into the coform web can actually improve the perceived softness and hand feel of the product while increasing surface durability.

Application of the additive composition also allows for the production of coform materials that have relatively low basis weight and/or have relatively high pulp content. For example, coform materials can be made having a basis weight of less than about 50 g/m$^2$, such as less than about 30 g/m$^2$. Coform materials made according to the present disclosure, for instance, can have a basis weight of from about 10 g/m$^2$ to about 50 g/m$^2$, such as from about 20 g/m$^2$ to about 50 g/m$^2$, such as from about 25 g/m$^2$ to about 30 g/m$^2$.

The coform materials can also contain greater amounts of pulp fibers. For instance, coform materials can be made containing pulp fibers in an amount greater than 70% by weight without having unacceptable lint properties. More particularly, the coform materials can contain pulp fibers in an amount from 70% by weight to 90% by weight or even greater. Increasing pulp content may increase the liquid absorption properties of the web and reduce the cost of producing the web.

Ultimately, reducing basis weight and/or increasing pulp content can increase production speeds.

The above advantages and benefits may be obtained by incorporating the additive composition into the base sheets at virtually any point during the manufacture of the sheet. The additive composition generally contains an aqueous dispersion comprising at least one thermoplastic resin, water, and, at least one dispersing agent. The thermoplastic resin is present within the dispersion at a relatively small particle size. For example, the average volumetric particle size of the polymer may be less than about 5 micrometers. actual particle size may depend upon various factors including the thermoplastic polymer that is present in the dispersion. Thus, the average volumetric particle size may be from about 0.05 micrometers to about 5 micrometers such as less than about 4 micrometers, such as less than about 3 micrometers, such as less than about 2 micrometers, such as less than about 1 micrometer. Particle sizes can be measured on a Coulter LS230 light-scattering particle size analyzer or other suitable device. When present in the aqueous dispersion

and when present in the tissue web, the thermoplastic resin is typically found in a non-fibrous form.

The particle size distribution of the polymer particles in the dispersion may be less than or equal to about 2.0, such as less than 1.9, 1.7 or 1.5.

Examples of aqueous dispersions that may be incorporated into the additive composition of the present disclosure are disclosed, for instance, in U.S. Patent Application Publication No. 2005/0100754, U.S. Patent Application Publication No. 2005/0192365, PCT Publication No. WO 2005/021638, and PCT Publication No. WO 2005/021622, United States Serial No. 11/303,002, United States Serial No. 11/304,490, United States Serial No. 11/303,036, United States Serial No. 11/304,998, United States Serial No. 11/304,063 and United States Serial No. 11/635,385.

In one embodiment, the additive composition may comprise a film forming composition capable of forming a film on the surface of a base sheet. For instance, when topically applied to a base sheet, the additive composition can form a discontinuous but interconnected film. In other words, the additive composition forms an interconnected polymer network over the surface of the base sheet. The film or polymer network, however, is discontinuous in that various openings are contained within the film. The size of the openings can vary depending upon the amount of additive composition that is applied to the sheet and the manner in which the additive composition is applied. Of particular advantage, the openings allow liquids to be absorbed through the discontinuous film and into the interior of the base sheet. In this regard, the wicking properties of the base sheet are not substantially affected by the presence of the additive composition.

In other embodiments, when the additive composition is added in relatively small amounts to the base sheet, the additive composition does not form an interconnected network but, instead, appears on the base sheet as treated discrete areas. Even at relatively low amounts, however, the additive composition can still enhance at least one property of the base sheet. For instance, the feel of the base sheet can be improved even in amounts less than about 2.5% by weight, such as less than 2% by weight, such as less than 1.5% by weight, such as less than 1% by weight, such as even less than 0.5% by weight.

Further, in some embodiments, the additive composition remains primarily on the surface of the base sheet and does not penetrate the sheet once applied. In this manner, not only does the discontinuous film allow the base sheet to absorb fluids that contact the surface but also does not significantly interfere with the ability of the base sheet to absorb relatively large amounts of fluid. Thus, the additive composition does not significantly interfere with the liquid absorption properties of the sheet while increasing the strength of the sheet without substantially impacting adversely on the stiffness of the sheet.

The thickness of the additive composition when present on the surface of a base sheet can vary depending upon the ingredients of the additive composition and the amount applied. In general, for instance, the thickness can vary from about 0.01 micrometers to about 10 micrometers. At higher add-on levels, for instance, the thickness may be from about 3 micrometers to about 8 micrometers. At lower add-on levels, however, the thickness may be from about 0.1 micrometers to about 1 micrometer, such as from about 0.3 micrometers to about 0.7 micrometers.

At relatively low add-on levels, the additive composition may also deposit differently on the base sheet than when at relatively high add-on levels. For example, at relatively low add-on levels, not only do discrete treated areas form on the base sheet, but the additive composition may better follow the topography of the base sheet. For instance, in one embodiment, it has been discovered that the additive composition follows the crepe pattern of a base sheet when the base sheet is creped.

The thermoplastic resin contained within the additive composition may vary depending upon the particular application and the desired result. In one embodiment, for instance, thermoplastic resin is an olefin polymer. As used herein, an olefin polymer refers to a class of unsaturated open-chain hydrocarbons having the general formula $C_nH_{2n}$. The olefin polymer may be present as a copolymer, such as an interpolymer. As used herein, a substantially olefin polymer refers to a polymer that contains less than about 1% substitution.

In one particular embodiment, for instance, the olefin polymer may comprise an alpha-olefin interpolymer of ethylene with at least one comonomer selected from the group consisting of a $C_4$-$C_{20}$ linear, branched or cyclic diene, or an ethylene vinyl compound, such as vinyl acetate, and a compound represented by the formula $H_2C$=CHR wherein R is a $C_1$-$C_{20}$ linear, branched or cyclic alkyl group or a $C_6$-$C_{20}$ aryl group. Examples of comonomers include propylene, 1-butene, 3-methyl-1-butene, 4-methyl-1-pentene, 3-methyl-1-pentene, 1-heptene, 1-hexene, 1-octene, 1-decene, and 1-dodecene. In some embodiments, the interpolymer of ethylene has a density of less than about 0.92 g/cm$^3$.

In other embodiments, the thermoplastic resin comprises an alpha-olefin interpolymer of propylene with at least one comonomer selected from the group consisting of ethylene, a $C_4$-$C_{20}$ linear, branched or cyclic diene, and a compound represented by the formula $H_2C$=CHR wherein R is a $C_1$-$C_{20}$ linear, branched or cyclic alkyl group or a $C_6$-$C_{20}$ aryl group. Examples of comonomers include ethylene, 1-butene, 3-methyl-1-butene, 4-methyl-1-pentene, 3-methyl-1-pentene, 1-heptene, 1-hexene, 1-octene, 1-decene, and 1-dodecene. In some embodiments, the comonomer is present at about 5% by weight to about 25% by weight of the interpolymer. In one embodiment, a propylene-ethylene interpolymer is used.

Other examples of thermoplastic resins which may be used in the present disclosure include homopolymers

and copolymers (including elastomers) of an olefin such as ethylene, propylene, 1-butene, 3-methyl-1-butene, 4-methyl-1-pentene, 3-methyl-1-pentene, 1-heptene, 1-hexene, 1-octene, 1-decene, and 1-dodecene as typically represented by polyethylene, polypropylene, poly-1-butene, poly-3-methyl-1-butene, poly-3-methyl-1-pentene, poly-4-methyl-1-pentene, ethylene-propylene copolymer, ethylene-1-butene copolymer, and propylene-1-butene copolymer; copolymers (including elastomers) of an alpha-olefin with a conjugated or non-conjugated diene as typically represented by ethylene-butadiene copolymer and ethylene-ethylidene norbornene copolymer; and polyolefins (including elastomers) such as copolymers of two or more alphaolefins with a conjugated or non-conjugated diene as typically represented by ethylene-propylene-butadiene copolymer, ethylene-propylene- dicyclopentadiene copolymer, ethylene-propylene-1,5-hexadiene copolymer, and ethylene-propylene-ethylidene norbornene copolymer; ethylene-vinyl compound copolymers such as ethylene-vinyl acetate copolymers with N-methylol functional comonomers, ethylene-vinyl alcohol copolymers with N-methylol functional comonomers, ethylene-vinyl chloride copolymer, ethylene acrylic acid or ethylene-(meth)acrylic acid copolymers, and ethylene-(meth)acrylate copolymer; styrenic copolymers (including elastomers) such as polystyrene, ABS, acrylonitrile-styrene copolymer, methylstyrene-styrene copolymer; and styrene block copolymers (including elastomers) such as styrene-butadiene copolymer and hydrate thereof, and styrene-isoprene-styrene triblock copolymer; polyvinyl compounds such as polyvinyl chloride, polyvinylidene chloride, vinyl chloride-vinylidene chloride copolymer, polymethyl acrylate, and polymethyl methacrylate; polyamides such as nylon 6, nylon 6,6, and nylon 12; thermoplastic polyesters such as polyethylene terephthalate and polybutylene terephthalate; polycarbonate, polyphenylene oxide, and the like. These resins may be used either alone or in combinations of two or more.

In particular embodiments, polyolefins such as polypropylene, polyethylene, and copolymers thereof and blends thereof, as well as ethylene-propylene-diene terpolymers are used. In some embodiments, the olefinic polymers include homogeneous polymers described in U.S. Pat. No. 3,645,992 by Elston; high density polyethylene (HDPE) as described in U.S. Pat. No. 4,076,698 to Anderson; heterogeneously branched linear low density polyethylene (LLDPE); heterogeneously branched ultra low linear density (ULDPE); homogeneously branched, linear ethylene/alpha-olefin copolymers; homogeneously branched, substantially linear ethylene/alpha-olefin polymers which can be prepared, for example, by a process disclosed in U.S. Pat. Nos. 5,272,236 and 5,278,272 ; and high pressure, free radical polymerized ethylene polymers and copolymers such as low density polyethylene (LDPE). In still another embodiment of the present invention, the thermoplastic resin comprises an ethylene-carboxylic acid copolymer, such as ethylene-acrylic acid (EAA) and ethylene-methacrylic acid copolymers such as for example those available under the tradenames PRIMACOR™ from The Dow Chemical Company, NUCREL™ from DuPont, and ESCOR™ from ExxonMobil, and described in U.S. Pat. Nos. 4,599,392, 4,988,781, and 5,384,373 ,and ethylene-vinyl acetate (EVA) copolymers. Polymer compositions described in U.S. Pat. Nos. 6,538,070, 6,566,446, 5,869,575, 6,448,341, 5,677,383, 6,316,549, 6,111,023, or 5,844,045 , are also suitable in some embodiments. Of course, blends of polymers can be used as well. In some embodiments, the blends include two different Ziegler-Natta polymers. In other embodiments, the blends can include blends of a Ziegler-Natta and a metallocene polymer. In still other embodiments, the thermoplastic resin used herein is a blend of two different metallocene polymers.

In one particular embodiment, the thermoplastic resin comprises an alpha-olefin interpolymer of ethylene with a comonomer comprising an alkene, such as 1-octene. The ethylene and octene copolymer may be present alone in the additive composition or in combination with another thermoplastic resin, such as ethylene-acrylic acid copolymer. Of particular advantage, the ethylene-acrylic acid copolymer not only is a thermoplastic resin, but also serves as a dispersing agent. For some embodiments, the additive composition should comprise a film-forming composition. It has been found that the ethylene-acrylic acid copolymer may assist in forming films, while the ethylene and octene copolymer lowers the stiffness. When applied to a base sheet, the composition may or may not form a film within the product, depending upon how the composition is applied and the amount of the composition that is applied. When forming a film on the base sheet, the film may be continuous or discontinuous. When present together, the weight ratio between the ethylene and octene copolymer and the ethylene-acrylic acid copolymer may be from about 1:10 to about 10:1, such as from about 3:2 to about 2:3.

The thermoplastic resin, such as the ethylene and octene copolymer, may have a crystallinity of less than about 50%, such as less than about 25%. The polymer may have been produced using a single site catalyst and may have a weight average molecular weight of from about 15,000 to about 5 million, such as from about 20,000 to about 1 million. The molecular weight distribution of the polymer may be from about 1.01 to about 40, such as from about 1.5 to about 20, such as from about 1.8 to about 10.

Depending upon the thermoplastic polymer, the melt index of the polymer may range from about 0.001 g/10 min to about 1,000 g/10 min, such as from about 0.5 g/10 min to about 800 g/10 min. For example, in one embodiment, the melt index of the thermoplastic resin may be from about 100 g/10 min to about 700 g/10 min.

The thermoplastic resin may also have a relatively low melting point. For instance, the melting point of the thermoplastic resin may be less than about 140°C, such as less than 130°C, such as less than 120°C. For instance, in one embodiment, the melting point may be less than about 90°C. The glass transition temperature of the thermoplastic resin may also be relatively low. For instance, the glass transition temperature may be less than about 50°C, such as

less than about 40C.

The one or more thermoplastic resins may be contained within the additive composition in an amount from about 1 % by weight to about 96% by weight. For instance, the thermoplastic resin may be present in the aqueous dispersion in an amount from about 10% by weight to about 70% by weight, such as from about 20% to about 50% by weight.

In addition to at least one thermoplastic resin, the aqueous dispersion also contains adispersing agent. A dispersing agent is an agent that aids in the formation and/or the stabilization of the dispersion. One or more dispersing agents may be incorporated into the additive composition.

In general, any suitable dispersing agent can be used. In one embodiment, for instance, the dispersing agent comprises at least one carboxylic acid, a salt of at least one carboxylic acid, or carboxylic acid ester or salt of the carboxylic acid ester. Examples of carboxylic acids useful as a dispersant comprise fatty acids such as montanic acid, stearic acid, oleic acid, and the like. In some embodiments, the carboxylic acid, the salt of the carboxylic acid, or at least one carboxylic acid fragment of the carboxylic acid ester or at least one carboxylic acid fragment of the salt of the carboxylic acid ester has fewer than 25 carbon atoms. In other embodiments, the carboxylic acid, the salt of the carboxylic acid, or at least one carboxylic acid fragment of the carboxylic acid ester or at least one carboxylic acid fragment of the salt of the carboxylic acid ester has 12 to 25 carbon atoms. In some embodiments, carboxylic acids, salts of the carboxylic acid, at least one carboxylic acid fragment of the carboxylic acid ester or its salt has 15 to 25 carbon atoms are preferred. In other embodiments, the number of carbon atoms is 25 to 60. Some examples of salts comprise a cation selected from the group consisting of an alkali metal cation, alkaline earth metal cation, or ammonium or alkyl ammonium cation.

In still other embodiments, the dispersing agent is selected from the group consisting of ethylene-carboxylic acid polymers, and their salts, such as ethylene-acrylic acid copolymers or ethylene-methacrylic acid copolymers.

In other embodiments, the dispersing agent is selected from alkyl ether carboxylates, petroleum sulfonates, sulfonated polyoxyethylenated alcohol, sulfated or phosphated polyoxyethylenated alcohols, polymeric ethylene oxide/propylene oxide/ethylene oxide dispersing agents, primary and secondary alcohol ethoxylates, alkyl glycosides and alkyl glycerides.

When ethylene-acrylic acid copolymer is used as a dispersing agent, the copolymer may also serve as a thermoplastic resin.

In one particular embodiment, the aqueous dispersion contains an ethylene and octene copolymer, ethylene-acrylic acid copolymer, and a fatty acid, such as stearic acid or oleic acid. The dispersing agent, such as the carboxylic acid, may be present in the aqueous dispersion in an amount from about 0.1 % to about 10% by weight.

In addition to the above components, the aqueous dispersion also contains water. Water may be added as tap water or as deionized water, if desired. The pH of the aqueous dispersion is generally less than about 12, such as from about 5 to about 11.5, such as from about 7 to about 11. The aqueous dispersion may have a solids content of less than about 75%, such as less than about 70%. For instance, the solids content of the aqueous dispersion may range from about 5% to about 60%. In general, the solids content can be varied depending upon the manner in which the additive composition is applied or incorporated into the tissue web. For instance, when incorporated into a base sheet during formation, such as by being added with an aqueous suspension of fibers, a relatively high solids content can be used. When topically applied such as by spraying or printing, however, a lower solids content may be used in order to improve processability through the spray or printing device.

While any method may be used to produce the aqueous dispersion, in one embodiment, the dispersion may be formed through a melt-kneading process. For example, the kneader may comprise a Banbury mixer, single-screw extruder or a multi-screw extruder. The melt-kneading may be conducted under the conditions which are typically used for melt-kneading the one or more thermoplastic resins.

In one particular embodiment, the process includes melt-kneading the components that make up the dispersion. The melt-kneading machine may include multiple inlets for the various components. For example, the extruder may include four inlets placed in series. Further, if desired, a vacuum vent may be added at an optional position of the extruder.

In some embodiments, the dispersion is first diluted to contain about 1 to about 3% by weight water and then, subsequently, further diluted to comprise greater than about 25% by weight water.

When treating base sheets in accordance with the present disclosure, the additive composition containing the aqueous polymer dispersion can be applied to the sheet topically or can be incorporated into the sheet by being pre-mixed with the fibers that are used to form the web. In one embodiment, the additive composition may be applied topically to the sheet during a creping process. For instance, in one embodiment, the additive composition may be sprayed onto the sheet or onto a heated dryer drum in order to adhere the sheet to the dryer drum. The sheet can then be creped from the dryer drum. When the additive composition is applied to the sheet and then adhered to the dryer drum, the composition may be uniformly applied over the surface area of the sheet or may be applied according to a particular pattern.

When topically applied to a base sheet, the additive composition may be sprayed onto the sheet, extruded onto the sheet, or printed onto the sheet. When extruded onto the sheet, any suitable extrusion device may be used, such as a slot-coat extruder or a meltblown dye extruder. When printed onto the sheet, any suitable printing device may be

used. For example, an inkjet printer or a rotogravure printing device may be used. In another embodiment, the additive composition may be applied to the sheet as a foam. The additive composition may be applied to one side of the sheet or may be applied to both sides of the sheet.

In one embodiment, the additive composition may be heated prior to or during application to a base sheet. Heating the composition can lower the viscosity for facilitating application. For instance, the additive composition may be heated to a temperature of from about 50°C to about 150°C.

As described above, in one embodiment, the additive composition is applied to a base sheet comprising a coform web. One process, for instance, of forming a coform web is illustrated in Fig. 1. Basically, the method of formation of the coform nonwoven web involves extruding a molten polymeric material through a die head 11 into fine streams and attenuating the streams by converging flows of high velocity, heated gas (usually air) supplied from nozzles 12 and 13 to break the polymer streams into discontinuous microfibers of small diameter. The die head preferably includes at least one straight row of extrusion apertures. In general, the resulting microfibers have an average fiber diameter of up to only about 10 with very few, if any, of the microfibers exceeding 10 micrometers in diameter. The average diameter of the microfibers is usually greater than about one micrometers, and is preferably within the range of about 2-6 micrometers, averaging about 5 micrometers. While the microfibers are predominately discontinuous, they generally have a length exceeding that normally associated with staple fibers.

The primary gas stream 10 is merged with a secondary gas stream 14 containing individualized wood pulp fibers so as to integrate the two different fibrous materials in a single step. The individualized wood pulp fibers typically have a length of about 0.5 to 10 millimeters and a length -2-maximum width ratio of about 10/1:400/1. A typical cross-section has an irregular width of 30 microns and a thickness of 5 microns. The integrated air stream is then directed onto a forming surface to air form the nonwoven fabric. In the configuration shown in Fig. 1, the secondary gas stream 14 is formed by a pulp sheet divellicating apparatus comprising a picker row 20 having picking teeth for divellicating pulp sheets 21 into individual fibers. The pulp sheets 21 are fed radially, that is, along a picker row radius, to the picker row 20 by means of rows 22. As the teeth on the picker row 20 divellicate the pulp sheets 21 into individual fibers, the resulting separated fibers are conveyed downwardly toward the primary air stream through a forming nozzle or duct **23.** A housing **24** encloses the picker row **20** and provides a passage **25** between the housing **24** and the picker row surface. Process air is supplied to the picker row in the passage **25** by means of duct **26** in sufficient quantity to serve as a medium for conveying the fibers through the forming duct **23** at a velocity approaching that of the picker teeth. The air may be supplied by any conventional means as, for example, a blower.

To convert the fiber blend in the integrated stream **15** into an integral fibrous mat or web, the stream **15** is passed into the nip of a pair of vacuum rolls **30** and **31** having foraminous surfaces that rotate continuously over a pair of thick vacuum nozzles **32** and **33.** As the integrated stream **15** enters the nip of the rolls **30** and **31,** the carrying gas is sucked into the two vacuum nozzles **32** and **33** while the fiber blend is supported and slightly compressed by the opposed surfaces of the two rolls **30** and **31.** This forms an integrated, self-supporting fibrous web **34** that has sufficient integrity to permit it to be withdrawn from the vacuum roll nip and conveyed to a windup roll **35.**

In accordance with the present disclosure, the additive composition may be applied at different locations in the process illustrated in **Fig. 1.** For instance, in one embodiment, the pulp fibers may be pretreated with the additive composition. Alternatively, the additive composition can be applied to the stream **15** prior to the vacuum rolls **30** and **31** or may be applied to the fibrous web **34.** If desired, the process can include a heating device for drying the additive composition prior to being wound into the roll **35.**

When applied to the stream **15** or to the fibrous web **34,** the additive composition can be applied to one side or can be applied to both sides of the stream or web. As described above, any suitable method may be used to apply the additive composition. For instance, the composition can be sprayed, extruded or printed onto the stream or the web.

In an alternative embodiment, the additive composition may be applied to the coform material through a creping process.

For example, in one embodiment, the additive composition may be used during a print-creping process and applied to a preformed web. Specifically, once topically applied to a coform web, the additive composition has been found well-suited to adhering the web to a creping surface, such as in a print-creping operation.

For example, once a coform web is formed, in one embodiment, the additive composition may be applied to at least one side of the web and then at least one side of the web may then be creped. In general, the additive composition may be applied to only one side of the web and only one side of the web may be creped, the additive composition may be applied to both sides of the web and only one side of the web is creped, or the additive composition may be applied to each side of the web and each side of the web may be creped.

Referring to Fig. 2, one embodiment of a system that may be used to apply the additive composition to a coform web and to crepe one side of the web is illustrated. The embodiment shown in **Fig. 2** can be an in-line or off-line process. As shown, coform web **80** made according to the process illustrated in **Fig. 1** according to a similar process, is passed through a first additive composition application station generally **82.** Station **82** includes a nip formed by a smooth rubber press roll **84** and a patterned rotogravure roll **86.** Rotogravure roll **86** is in communication with a reservoir **88** containing

a first additive composition **90.** Rotogravure roll **86** applies the additive composition 90 to one side of web **80** in a preselected pattern.

Web **80** is then contacted with a heated roll **92** after passing a roll **94.** The heated roll **92** can be heated to a temperature, for instance, up to about 200°C and particularly from about 100°C to about 150°C. In general, the web can be heated to a temperature sufficient to dry the web and evaporate any water.

It should be understood, that besides the heated roll **92,** any suitable heating device can be used to dry the web. For example, in an alternative embodiment, the web can be placed in communication with an infra-red heater in order to dry the web. Besides using a heated roll or an infra-red heater, other heating devices can include, for instance, any suitable convective oven or microwave oven.

From the heated roll **92,** the web **80** can be advanced by pull rolls **96** to a second additive composition application station generally **98.** Station **98** includes a transfer roll **100** in contact with a rotogravure roll **102,** which is in communication with a reservoir **104** containing a second additive composition **106.** Similar to station **82,** second additive composition **106** is applied to the opposite side of web **80** in a preselected pattern. Once the second additive composition is applied, web **80** is adhered to a creping roll **108** by a press roll **110.** Web **80** is carried on the surface of the creping drum **108** for a distance and then removed therefrom by the action of a creping blade **112.** The creping blade **112** performs a controlled pattern creping operation on the second side of the web.

Once creped, coform web **80,** in this embodiment, is pulled through a drying station **114.** Drying station **114** can include any form of a heating unit, such as an oven energized by infra-red heat, microwave energy, hot air or the like. Drying station **114** may be necessary in some applications to dry the web and/or cure the additive composition. Depending upon the additive composition selected, however, in other applications drying station **114** may not be needed.

The amount that the coform web is heated within the drying station **114** can depend upon the particular thermoplastic resins used in the additive composition, the amount of the composition applied to the web, and the type of web used. In some applications, for instance, the coform web can be heated using a gas stream such as air at a temperature of about 100°C to about 200°C.

In the embodiment illustrated in **Fig. 2,** although the additive composition is being applied to each side of the tissue web, only one side of the web undergoes a creping process. It should be understood, however, that in other embodiments both sides of the web may be creped. For instance, the heated roll 92 may be replaced with a creping drum such as **108** shown in **Fig. 2.**

Creping the base sheet as shown in **Fig. 2** increases the softness of the web by breaking apart fiber-to-fiber bonds contained within the web. Applying the additive composition to the outside of the web, on the other hand, not only assists in creping the web but also adds dry strength, wet strength, stretchability and tear resistance to the web. Further, the additive composition reduces the release of lint from the web.

In addition, applying the additive composition to the coform web and creping the web creates soft ripples on the side of the sheet that is creped. If only one side of the sheet is creped, the resulting material has different textures on each surface. If both sides of the material are creped, on the other hand, both sides will have ripples. The ripples may lower capillary tension with regard to a wipe solution that may be applied to the sheet. When forming ripples as described above, it may not be desirable to emboss or calender the web after formation.

In general, the first additive composition and the second additive composition applied to the coform web as shown in **Fig. 2** may contain the same ingredients or may contain different ingredients. Alternatively, the additive compositions may contain the same ingredients in different amounts as desired.

The additive composition is applied to the base web as described above in a preselected pattern. In one embodiment, for instance, the additive composition can be applied to the web in a reticular pattern, such that the pattern is interconnected forming a net-like design on the surface.

In an alternative embodiment, however, the additive composition is applied to the web in a pattern that represents a succession of discrete shapes. Applying the additive composition in discrete shapes, such as dots, provides sufficient strength to the web without covering a substantial portion of the surface area of the web.

According to the present disclosure, the additive composition is applied to each side of the base sheet so as to cover from about 15% to about 99% of the surface area of the web. More particularly, in most applications, the additive composition will cover from about 20% to about 60% of the surface area of each side of the web. The total amount of additive composition applied to each side of the web can be in the range of from about 0.5% to about 30% by weight, based upon the total weight of the web, such as from about 1% to about 20% by weight, such as from about 2% to about 10% by weight.

In one embodiment, the amount of additive composition applied to the coform web can depend upon the basis weight of the web. For instance, if the basis weight of the web is greater than about 50 $g/m^2$, the additive composition can be applied in an amount from about 0.2 $g/m^2$ about 1 $g/m^2$ For lower basis weights, such as less than about 50 $g/m^2$, on the other hand, greater amounts of the additive composition can be applied to the web. For instance, when the basis weight of the web is less than about 50 $g/m^2$, the additive composition can be applied in amounts greater than 1 $g/m^2$, such as from about 1 $g/m^2$ to about 5 $g/m^2$. At lower basis weights or for coform webs containing lower amounts

of synthetic fibers, greater amounts of the additive composition can be applied in order to increase strength and reduce lint.

At the above amounts, the additive composition can penetrate the tissue web after being applied in an amount up to about 30% of the total thickness of the web, depending upon various factors. It has been discovered, however, that most of the additive composition primarily resides on the surface of the web after being applied to the web. For instance, in some embodiments, the additive composition penetrates the web less than 5%, such as less than 3%, such as less than 1% of the thickness of the web.

Referring to **Fig. 3,** one embodiment of a pattern that can be used for applying an additive composition to a base sheet in accordance with the present disclosure is shown. As illustrated, the pattern shown in **Fig. 3** represents a succession of discrete dots **120.** In one embodiment, for instance, the dots can be spaced so that there are approximately from about 25 to about 35 per 2.54-cm dots(per inch) in the machine direction or the cross-machine direction. The dots can have a diameter, for example, of from about 0.25 mm (about 0.01 inches) to about 0.76 mm about 0.03 inches). In one particular embodiment, the dots can have a diameter of about 0.51 mm (about 0.02 inches)and can be present in the pattern so that approximately 28 dots per 2.54 cm (per inch) extend in either the machine direction or the cross-machine direction. In this embodiment, the dots can cover from about 20% to about 30% of the surface area of one side of the base sheet and, more particularly, can cover about 25% of the surface area of the web.

Besides dots, various other discrete shapes can also be used. For example, as shown in **Fig. 5,** a pattern is illustrated in which the pattern is made up of discrete shapes that are each comprised of three elongated hexagons. In one embodiment, the hexagons can be about 0.51mm (about 0.02 inches) long and can have a width of about 0.15 mm (about 0.006 inches). Approximately 35 to 40 hexagons per 2.54 cm (per inch) can be spaced in the machine direction and the cross-machine direction. When using hexagons as shown in **Fig. 5,** the pattern can cover from about 40% to about 60% of the surface area of one side of the web, and more particularly can cover about 50% of the surface area of the web.

Referring to **Fig. 4,** another embodiment of a pattern for applying an additive composition to a base sheet is shown. In this embodiment, the pattern is a reticulated grid. More specifically, the reticulated pattern is in the shape of diamonds. When used, a reticulated pattern may provide more strength to the web in comparison to patterns that are made up on a succession of discrete shapes.

The process that is used to apply the additive composition to the base sheet in accordance with the present disclosure can vary. For example, various printing methods can be used to print the additive composition onto the base sheet depending upon the particular application. Such printing methods can include direct gravure printing using two separate gravures for each side, offset gravure printing using duplex printing (both sides printed simultaneously) or station-to-station printing (consecutive printing of each side in one pass). In another embodiment, a combination of offset and direct gravure printing can be used. In still another embodiment, flexographic printing using either duplex or station-to-station printing can also be utilized to apply the additive composition.

Coform base sheets made according to the present disclosure can have relatively good bulk characteristics. For instance, the coform webs can have a bulk greater than about 3 $cm^3/g$, such as greater than about 5 $cm^3/g$, such as greater than about 7 $cm^3/g$, such as greater than about 9 $cm^3/g$, such as even greater than about 10 $cm^3/g$. The basis weight of the coform webs can vary from about 15 $g/m^2$ to 200 $g/m^2$ or greater. As described above, in one embodiment, coform webs are made having a relatively low basis weight, such as less than about 50 $g/m^2$.

Referring to **Fig. 6,** one embodiment of a process for applying the additive composition to only one side of a base sheet in accordance with the present disclosure is shown. The process illustrated in **Fig. 6** is similar to the process shown in **Fig. 2.** In this regard, like reference numerals have been used to indicate similar elements.

As shown, a web **80** is advanced to an additive composition application station generally **98.** Station **98** includes a transfer roll **100** in contact with a rotogravure roll **102,** which is in communication with a reservoir **104** containing an additive composition **106.** At station **98,** the additive composition **106** is applied to one side of the web **80** in a preselected pattern.

Once the additive composition is applied, web **80** is adhered to a creping roll **108** by a press roll **110.** Web **80** is carried on the surface of the creping drum **108** for a distance and then removed therefrom by the action of a creping blade **112.** The creping blade **112** performs a controlled pattern creping operation on the treated side of the web.

From the creping drum **108,** the coform web **80** is fed through a drying station **114** which dries and/or cures the additive composition **106.** The web **80** is then wound into a roll **116** for use in forming multiple ply products or a single ply product.

Referring to **Fig. 7,** another embodiment of a process for applying the additive composition to only one side of a tissue web in accordance with the present disclosure is shown. Like reference numerals have been used to indicate similar elements.

The process illustrated in **Fig. 7** is similar to the process illustrated in **Fig. 6.** In the process shown in **Fig. 37,** however, the additive composition is indirectly applied to the base sheet **80** by an offset printing apparatus in an offset printing arrangement.

For instance, as shown in **Fig. 7,** the additive composition **106** is first transferred to a first print roll **102.** From

the print roll **102,** the additive composition is then transferred to an analog roll **103** prior to being applied to the base sheet **80.** From the analog roll **103,** the additive composition is pressed onto the web **80** through the assistance of a rubber backing roll **100.**

Similar to **Fig. 6,** once the additive composition is applied to the web **80,** the web is then adhered to a heated creping drum **108** and creped from the drum using a creping blade **112** prior to being wound into a roll **116.**

Referring to **Fig. 8,** still another embodiment of a process for applying the additive composition to only one side of the base sheet in accordance with the present disclosure is illustrated. As shown, in this embodiment, a formed web **80** is unwound from a roll **85** and fed into the process. This process may be considered an off-line process, although the application method may also be installed in-line.

As illustrated in **Fig. 8,** the web **80** is pressed against a dryer drum **108** by a press roll **110.** A spray device **109** applies the additive composition of the present disclosure to the surface of the dryer drum. The additive composition thus not only adheres the web **80** to the surface of the dryer drum **108,** but also transfers to the web as the web is creped from the drum using a creping blade **112.** Once creped from the dryer drum **108,** the web **80** is wound into a roll **116.**

The embodiment illustrated in **Fig. 8** may be considered a spray crepe process. During the process, the dryer drum **108** can be heated to temperatures as described above with respect to the other embodiments illustrated in the figures.

When only treating one side of the base sheet **80** with an additive composition, in one embodiment, it may be desirable to apply the additive composition according to a pattern that covers greater than about 40% of the surface area of one side of the web. For instance, the pattern may cover from about 40% to about 90% of the surface area of one side of the web such as from about 40% to about 60%. In one particular example, for instance, the additive composition can be applied according to the pattern shown in **Fig. 5.**

As described above, base sheets made in accordance with the present disclosure may be incorporated into one-ply products or may be incorporated into products having multiple plies. For instance, in one embodiment, coform webs treated in accordance with the present disclosure may be combined together to form a laminate. For example, in one embodiment, a two-ply product can be formed from coform webs made according to the process shown in **Fig. 6.** When combined together, the creped sides of the webs may form the exterior surfaces of the resulting product.

The manner in which the first web is laminated to the second web may vary depending upon the particular application and desired characteristics. In some applications, the alpha-olefin interpolymer of the present disclosure may serve as the ply-bonding agent. In other applications, a binder material, such as an adhesive or binder fibers, is applied to one or both webs to join the webs together. The adhesive can be, for instance, a latex adhesive, a starch-based adhesive, an acetate such as an ethylene-vinyl acetate adhesive, a polyvinyl alcohol adhesive, and the like. It should be understood, however, that other binder materials, such as thermoplastic films and fibers can also be used to join the webs. In still an alternative embodiment, two coform webs may be bonded together through thermal bonding or ultrasonic bonding.

In one embodiment, a coform web made in accordance with the present disclosure may be bonded to a tissue web. Bonding a coform web to a tissue web provides a product having a dual texture on each side. In particular, a product is formed having two distinct wiping surfaces with different pore structures and topography.

In general, any suitable tissue web may be bonded to the coform web. The tissue web, for instance, may contain pulp fibers, such as softwood fibers and/or hardwood fibers, in an amount greater than 80% by weight, such as in an amount greater than 90% by weight. The tissue web may be formed according to an airlaid process or a wet lay process. The tissue web, for instance, can be creped, double creped, embossed, wet pressed, air pressed, through-air dried, creped through-air dried, uncreped through-air dried, and the like.

The tissue web may have a basis weight of from about 10 $g/m^2$ to about 110 $g/m^2$, and can have a bulk of from about 3 $cm^3/g$ to about 20 $cm^3/g$, such as from about 5 $cm^3/g$ to about 15 $cm^3/g$.

In one embodiment, the tissue web may be pattern densified or imprinted such as the tissue sheets disclosed in any of the following U.S. Patent Nos.: 4,514,345 issued on April 30, 1985, to Johnson et al.; 4,528,239 issued on July 9, 1985, to Trokhan; 5,098,522 issued on March 24, 1992; 5,260,171 issued on November 9, 1993, to Smurkoski et al. ; 5,275,700 issued on January 4, 1994, to Trokhan; 5,328,565 issued on July 12, 1994, to Rasch et al.; 5,334,289 issued on August 2, 1994, to Trokhan et al.; 5,431,786 issued on July 11, 1995, to Rasch et al.; 5,496,624 issued on March 5, 1996, to Steltjes, Jr. et al.; 5,500,277 issued on March 19, 1996, to Trokhan et al.; 5,514,523 issued on May 7, 1996, to Trokhan et al.; 5,554,467 issued on September 10, 1996, to Trokhan et al.; 5,566,724 issued on October 22, 1996, to Trokhan et al.; 5,624,790 issued on April 29, 1997, to Trokhan et al.; and, 5,628,876 issued on May 13, 1997, to Ayers et al. Such imprinted tissue sheets may have a network of densified regions that have been imprinted against a drum dryer by an imprinting fabric, and regions that are relatively less densified (e.g., "domes" in the tissue sheet) corresponding to deflection conduits in the imprinting fabric, wherein the tissue sheet superposed over the deflection conduits was deflected by an air pressure differential across the deflection conduit to form a lower-density pillow-like region or dome in the tissue sheet.

In an alternative embodiment, the tissue web is a textured web which has been dried in a three-dimensional

state such that the hydrogen bonds joining fibers were substantially formed while the web was not in a flat, planar state. For instance, the web can be formed while the web is on a highly textured throughdrying fabric or other three-dimensional substrate. Processes for producing uncreped throughdried fabrics are, for instance, disclosed in U. S. Patent No. 5,672,248 to Wendt, et al.; U. S. Patent No. 5,656,132 to Farrington, et al.; U. S. Patent No. 6,120,642 to Lindsay and Burazin; U.S. Patent No. 6,096,169 to Hermans, et al.; U. S. Patent No. 6,197,154 to Chen. et al.; and U. S. Patent No. 6,143,135 to Hada, et al.

Once formed, the products may be packaged in different ways. For instance, in one embodiment, the treated sheet products may be cut into individual sheets and stacked prior to being placed into a package. Alternatively, the sheet products may be spirally wound. When spirally wound together, each individual sheet may be separated from an adjacent sheet by a line of weakness, such as a perforation line.

The coform materials made in accordance with the present disclosure may be used to form numerous products. For instance, in one embodiment, the coform materials may be used to form wiping products, such as industrial wipers, napkins, towels, and the like. The coform material may also be incorporated into personal absorbent products, such as a liner in a diaper, pull-up, feminine care product, or adult incontinence product. In still another embodiment, the coform material may be used in medical applications as a bandage, as a drape, or as any other suitable product.

The coform material may be used in a dry state or in a wet state. In one embodiment, for instance, the coform material may be impregnated with a wiping solution.

The wiping solution can comprise any suitable solution that will not degrade the base sheet.

For example, when used as a baby wipe, for instance, the wiping solution may contain water, one or more surfactants, and/or an emollient. The solution may also contain, for instance, one or more glycols. Examples of glycols include propylene glycol, or polyethylene glycol. Various other ingredients may also be incorporated into the wiping solution such as fragrances, aloe, and the like. The wiping solution may be alcohol-free or may contain an alcohol.

When used to clean adjacent surfaces, for instance, the wiping solution may contain one or more alcohols combined with water. The alcohol may be, for instance, an aliphatic alcohol having from about 1 to about 6 carbon atoms. By way of example, the alcohol may be methanol, ethanol, propanol, isopropanol, butanol, t-butanol, 2-butanol, pentanol, 2-pentanol, hexanol, 2,3-dimethyl-1-butanol, and the like, including mixtures of two or more alcohols.

In general, the wiping solution can contain water in an amount less than about 50% by weight. For instance, in one embodiment, the solution may contain alcohol in an amount greater than about 60% by weight, such as from about 60% by weight to about 80% by weight. Greater amounts of alcohol, however, may be used.

The wiping solution may also contain various other additives. Such other additives include disinfectants, antiseptics, emollients, skin conditioners, anti-microbial agents such as sterilants, sporicides, germicides, bactericides, fungicides, virucides, protozoacides, algicides, bacteriostats, fungistats, virustats, sanitizers, and antibiotics, fragrances, anti-drying agents, and the like.

Example of anti-drying agents include glycols and glycerides. Examples of anti-microbial agents, on the other hand, include quaternary ammonium compounds, such as quaternary ammonium halide compounds. In some embodiments, quaternary ammonium halide compounds having the following formula are utilized:

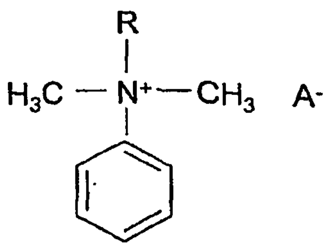

wherein,

R is a $C_8$-$C_{18}$ alkyl group; and

A is a halogen atom, such as chlorine, bromine, fluorine, and the like.

One commercially available example of an antimicrobial agent that includes such a quaternary ammonium compound is available under the trade name BARDAC® 208M from Lonza, Inc., Fairlawn, N.J. Specifically, BARDAC® 208M contains a blend of alkyl dimethyl benzyl ammonium chlorides. Other commercially available examples of suitable quaternary ammonium compounds are believed to include BARDAC® 2050 and BARDAC® 2080 (based on dialkyl($C_8$-$C_{10}$)dimethyl ammonium chloride); BARDAC® 2250 and BARDAC® 2280 (didecyl dimethyl ammonium chloride); BARDAC® LF and BARDAC® LF 80 (based on dioctyl dimethyl ammonium chloride); BARQUAT® MB-50 and BARQUAT® MB-80 (based on alkyl dimethyl benzyl ammonium chloride); BARQUAT® MX-50 and BARQUAT® MX-80 (based on alkyl dimethyl benzyl ammonium chloride); BARQUAT® OJ-50 and BARQUAT® OJ-80 (based on alkyl dimethyl benzyl ammonium chloride); BARQUAT® 4250, BARQUAT® 4280, BARQUAT® 4250Z, and BARQUAT®

4280Z (based on alkyl dimethyl benzyl ammonium chloride and/or alkyl dimethyl ethyl benzyl ammonium chloride); and BARQUAT® MS-100 (based on myristyl dimethyl benzyl ammonium chloride), which are available from Lonza, Inc., Fairlawn, N.J.

Other anti-microbial agents that may be used in the present disclosure include halogenated diphenyl ethers like 2,4,4'-trichloro-2'-hydroxy-diphenyl ether (Triclosan® or TCS) or 2,2'-dihydroxy-5,5'-dibromo-diphenyl ether; phenolic compounds like phenoxyethanol, phenoxy propanol, phenoxyisopropanol, para-chloro-meta-xylenol (PCMX), etc.; bisphenolic compounds like 2,2'-methylene bis (4-chlorophenol), 2,2'-methylene bis (3,4,6-trichlorophenol), 2,2'-methylene bis (4-chloro-6-bromophenol), bis (2-hydroxy-3,5-dichlorophenyl) sulphide, and bis (2-hydroxy-5-chlorobenzyl) sulphide; halogenated carbanilides (e.g., 3,4,4'-trichlorocarbanilides (Triclocarban® or TCC); benzyl alcohols; chlorhexidine; chlorhexidine gluconate; and chlorhexidine hydrochloride.

The wiping solution impregnated into the base sheet may also contain one or more surfactants. Surfactants can provide a number of benefits to the resulting wiper. For instance, surfactants can increase the wettability of the wiping product, can serve as emollients, can improve the ability of the wiping product to clean surfaces, and can also serve to stabilize the wiping solution itself. In general, any suitable nonionic, anionic, cationic and amphoteric surfactant may be incorporated into the wiping solution.

In some embodiments, the wiping solution can also contain one or more preservatives. Suitable preservatives include, for instance, Kathon CG®, which is a mixture of methylchloroisothiazolinone and methylisothiazolinone available from Rohm & Haas; Mackstat H 66 (available from McIntyre Group, Chicago, IL); CANGARD available from Dow Chemical, which is a benzisothiazoline; DMDM hydantoin (e.g., Glydant Plus, Lonza, Inc., Fair Lawn, NJ); iodopropynyl butylcarbamate; benzoic esters (parabens), such as methylparaben, propylparaben, butylparaben, ethylparaben, isopropylparaben, isobutylparaben, benzylparaben, sodium methylparaben, and sodium propylparaben; 2-bromo-2-nitropropane-1,3-diol; benzoic acid; amidazolidinyl urea; diazolidinyl urea; and the like. Other suitable preservatives include those sold by Sutton Labs, such as "Germall 115" (amidazolidinyl urea), "Germall II" (diazolidinyl urea), and "Germall Plus" (diazolidinyl urea and iodopropynyl butylcarbonate).

In general, any of the above additives may be present in the wiping solution in an amount less than about 20% by weight, such as less than about 5% by weight. For instance, many of the additives may be present in an amount from about 0.001 % to about 2% by weight.

Once the base sheet is impregnated with a wiping solution, the wiping products may be packaged as desired. For instance, the wiping product may be packaged in a resealable container. Some examples of suitable containers include rigid tubs, film pouches, etc. One particular example of a suitable container for holding the wipers is a rigid, cylindrical tub (e.g., made from polyethylene) that is fitted with a resealable air-tight lid on the top portion of the container. The lid has a hinged cap initially covering an opening positioned beneath the cap. The opening allows for the passage of wipers from the interior of the sealed container whereby individual wipers can be removed by grasping the wiper.

In another embodiment, the wiper may be held in a liquid impermeable pouch that has an ovular shaped opening. The opening may be covered by a tab that is attached to the pouch by a pressure sensitive adhesive. The tab may be opened to remove a wiper and then resealed against the pouch.

As described above, the additive composition can be applied to one side of a wiping product or can be applied to both sides of the wiping product. In one embodiment, the amount the additive composition is applied to each side can differ. For instance, greater amounts of the additive composition can be applied to a first side of a base sheet, while lesser amounts may be applied to the opposite side. In this manner, a wiping product can be produced that has different functions on each side. The side containing greater amounts of the additive composition may be used for heavier cleaning while the other side containing lesser amounts or being untreated can be used for shining or polishing an object.

Wiping products made according to the present disclosure can be used in numerous applications. For instance, dry wiping products may be used along with aqueous solutions such as cleaning solutions or cleaning aerosols. The wipers may be used for window cleaning, general purpose cleaning, makeup removal, skin cleansing, spa treatments, and the like. The wipers may also be used to polish furniture or can be used as any suitable type of health and beauty aid.

In one embodiment, base sheets made according to the present disclosure may be attached to a cleaning instrument for cleaning adjacent surfaces. For instance, in one embodiment, the base sheet comprises a disposable sheet that is to be used in conjunction with a mop head. The cleaning instrument can then be used to clean or polish floors. In one embodiment, a base sheet made in accordance with the present disclosure can be fastened around a sponge mop to pick up heavy dirt first. The base sheet can then be discarded and the mop used to clean the floor with a cleaning solution.

The present disclosure may be better understood with reference to the following examples.

EXAMPLE 1

In this example, tissue webs treated with an additive composition of the present disclosure were compared to commercially available products. The samples were subjected to various tests. In particular, the samples were subjected

to a "Stick-Slip Parameter Test" which measures the perceived softness of the product by measuring the spacial and temporal variation of a drag force as skin simulant is dragged over the surface of the sample. This example demonstrates how the additive composition improves the softness of base sheets.

More particularly, the following tests were performed in this example.

Stick-Slip Test

Stick-Slip occurs when the static coefficient of friction ("COF") is significantly higher than the kinetic COF. A sled pulled over a surface by a string will not move until the force in the string is high enough to overcome the static COF times the normal load. However, as soon as the sled starts to move the static COF gives way to the lower kinetic COF, so the pulling force in the string is unbalanced and the sled accelerates until the tension in the string is released and the sled stops (sticks). The tension then builds again until it is high enough to overcome the static COF, and so on. The frequency and amplitude of the oscillations depend upon the difference between the static COF and the kinetic COF, but also upon the length and stiffness of the string (a stiff, short string will let the force drop down almost immediately when the static COF is overcome so that the sled jerks forward only a small distance), and upon the speed of travel. Higher speeds tend to reduce Stick-Slip behavior.

Static COF is higher than kinetic COF because two surfaces in contact under a load tend to creep and comply with each other and increase the contact area between them. COF is proportional to contact area so more time in contact gives a higher COF. This helps explain why higher speeds give less Stick-Slip: there is less time after each slip event for the surfaces to comply and for the static COF to rise. For many materials the COF decreases with higher speed sliding because of this reduced time for compliance. However, some materials (typically soft or lubricated surfaces) actually show an increase in COF with increasing speed because the surfaces in contact tend to flow either plastically or viscoelastically and dissipate energy at a rate proportional to the rate at which they are sheared. Materials which have increasing COF with velocity do not show Stick-Slip because it would take more force to make the sled jerk forward than to continue at a constant slower rate. Such materials also have a static COF equal to their kinetic COF. Therefore, measuring the slope of the COF versus velocity curve is a good means of predicting whether a material is likely to show Stick-Slip: more negative slopes will Stick-Slip easily, while more positive slopes will not Stick-Slip even at very low velocities of sliding.

According to the Stick-Slip Test, the variation in COF with velocity of sliding is measured using an Alliance RT/1 tensile frame equipped with MTS TestWorks 4 software. A diagram of part of the testing apparatus is shown in Fig. 9. As illustrated, a plate is fixed to the lower part of the frame, and_a tissue sheet (the sample) is clamped to this plate. An aluminum sled with a 3.81 cm x 3.81 cm (1.5" by 1.5") flat surface With a 1.27 cm (½") radius on the leading and trailing edges is attached to the upper (moving part) of the frame by means of a slender fishing line (30 lb, Stren clear monofilament from Remington Arms Inc, Madison, NC) lead though a nearly frictionless pulley up to a 50 N load cell. A 50.8 mm wide sheet of collagen film is clamped flat to the underside of the sled by means of 32 mm binder clips on the front and back of the sled. The total mass of the sled, film and clips is 81.1 g. The film is larger than the sled so that it fully covers the contacting surfaces. The collagen film may be obtained from NATURIN GmbH, Weinhein, Germany, under the designation of COFFI (Collagen Food Film), having a basis weight of 28 g/m$^2$. Another suitable film may be obtained from Viscofan USA Inc, 50 County Court, Montgomery AL 36105. The films are embossed with a small dot pattern. The flatter side of the film (with the dots dimpled down) should be facing down toward the tissue on the sled to maximize contact area between the tissue and collagen. The samples and the collagen film should be conditioned at 22.2 °C (72°F) and 50% RH for at least 6 hours prior to testing.

The tensile frame is programmed to drag the sled at a constant velocity (V) for a distance of 1 cm while the drag force is measured at a frequency of 100 hz. The average drag force measured between 0.2 cm and 0.9 cm is calculated, and kinetic COF is calculated as:

$$COF_V = \frac{f}{81.1} \qquad (1)$$

Where f is the average drag force in grams, and 81.1 g is the mass of the sled, clips and film.

For each sample the COF is measured at 5, 10, 25, 50 and 100 cm/min. A new piece of collagen film is used for each sample.

The COF varies logarithmically with velocity, so that the data is described by the expression:

$$COF = a + SSP\ln(V)$$

Where a is the best fit COF at 1 cm/min and SSP is the Stick-Slip Parameter, showing how the COF varies with velocity. A higher value of SSP indicates a more lotiony, less prone to Stick-Slip sheet. SSP is measured for four tissue sheet samples for each code and the average is reported.

Hercules Size Test (HST)

The "Hercules Size Test" (HST) is a test that generally measures how long it takes for a liquid to travel through a tissue sheet. Hercules size testing was done in general accordance with TAPPI method T 530 PM-89, Size Test for Paper with Ink Resistance. Hercules Size Test data was collected on a Model HST tester using white and green calibration tiles and the black disk provided by the manufacturer. A 2% Napthol Green N dye diluted with distilled water to 1 % was used as the dye. All materials are available from Hercules, Inc., Wilmington, Delaware.

All specimens were conditioned for at least 4 hours at 23+/-1 °C and 50 +/-2% relative humidity prior to testing. The test is sensitive to dye solution temperature so the dye solution should also be equilibrated to the controlled condition temperature for a minimum of 4 hours before testing.

Six (6) tissue sheets as commercially sold (18 plies for a 3-ply tissue product, 12 plies for a two-ply product, 6 plies for a single ply product etc.) form the specimen for testing. Specimens are cut to an approximate dimension of 6.35 cm x 6.35 cm (2.5 X 2.5 inches). The instrument is standardized with white and green calibration tiles per the manufacturer's directions. The specimen (12 plies for a 2-ply tissue product) is placed in the sample holder with the outer surface of the plies facing outward. The specimen is then clamped into the specimen holder. The specimen holder is then positioned in the retaining ring on top of the optical housing. Using the black disk, the instrument zero is calibrated. The black disk is removed and 10 +/-0.5 milliliters of dye solution is dispensed into the retaining ring and the timer started while placing the black disk back over the specimen. The test time in seconds (sec.) is recorded from the instrument.

Extraction Method for Determining Additive Content in Tissue

One method for measuring the amount of additive composition in a tissue sample is removal of the additive composition in a suitable solvent. Any suitable solvent may be selected, provided that it can dissolve at least a majority of the additive present in the tissue. One suitable solvent is Xylene.

To begin, a tissue sample containing the additive composition (3 grams of tissue minimum per test) was placed in an oven set at 105°C overnight to remove all water. The dried tissue was then sealed in a metal can with a lid and allowed to cool in a dessicator containing calcium sulfate dessicant to prevent absorption of water from the air. After allowing the sample to cool for 10 minutes, the weight of the tissue was measured on a balance with an accuracy of $\pm$0.0001 g. and the weight recorded ($W_1$).

The extraction was performed using a soxhlet extraction apparatus. The soxhlet extraction apparatus consisted of a 250 ml glass round bottom flask connected to a soxhlet extraction tube (Corning® no. 3740-M, with a capacity to top of siphon of 85 ml) and an Allihn condenser (Corning® no. 3840-MCO). The condenser was connected to a fresh cold water supply. The round bottom flask was heated from below using an electrically heated mantle (Glas Col, Terre Haute, IN USA) controlled by a variable auto transformer (Superior Electric Co., Bristol, CT USA).

To conduct an extraction, the pre-weighed tissue containing the additive composition was placed into a 33 mm x 80 mm cellulose extraction thimble (Whatman International Ltd, Maidstone, England). The thimble was then put into the soxhlet extraction tube and the tube connected to the round bottom flask and the condenser. Inside the round bottom flask was 150 ml of xylene solvent. The heating mantle was energized and water flow through the condenser was initiated. The variable auto transformer heat control was adjusted such that the soxhlet tube filled with xylene and cycled back into the round bottom flask every 15 minutes. The extraction was conducted for a total of 5 hours (approximately 20 cycles of xylene through the soxhlet tube). Upon completion the thimble containing the tissue was removed from the soxhlet tube and allowed to dry in a hood. The tissue was then transported to an oven set at 150°C and dried for 1 hour to remove excess xylene solvent. This oven was vented to a hood. The dry tissue was then placed in an oven set at 105°C overnight. The next day the tissue was removed, placed in a metal can with a lid, and allowed to cool in a desiccator containing calcium sulfate desiccant for 10 minutes. The dry, cooled extracted tissue weight was then measured on a balance with an accuracy of +0.0001 g. and the weight recorded ($W_2$).

The % xylene extractives was calculated using the equation below:

$$\%\ \text{xylene extractives} = 100 \times (W_1 - W_2) \div W_1$$

Because not all of the additive composition may extract in the selected solvent, it was necessary to construct a calibration curve to determine the amount of additive composition in an unknown sample. A calibration curve was

developed by first applying a known amount of additive to the surface of a pre-weighed tissue ($T_1$) using an air brush. The additive composition was applied evenly over the tissue and allowed to dry in an oven at 105°C overnight. The weight of the treated tissue was then measured ($T_2$) and the weight % of additive was calculated using the equation below:

$$\% \text{ additive} = 100 \times (T_2 - T_1) \div T_1$$

Treated tissues over a range of additive composition levels from 0% to 13% were produced and tested using the soxhlet extraction procedure previously described. The linear regression of % xylene extractives (Y variable) vs. % additive (X variable) was used as the calibration curve.

$$\text{Calibration curve:} \quad \% \text{ xylene extractives} = m(\% \text{ additive}) + b$$

or:

$$\% \text{ additive} = (\% \text{ xylene extractives} - b) / m$$

where:

m = slope of linear regression equation
b = y-intercept of linear regression equation

After a calibration curve has been established, the additive composition of a tissue sample can be determined. The xylene extractives content of a tissue sample was measured using the soxhlet extraction procedure previously described. The % additive in the tissue was then calculated using the linear regression equation:

$$\% \text{ additive} = (\% \text{ xylene extractives} - b) / m$$

where:

m = slope of linear regression equation
b = y-intercept of linear regression equation

A minimum of two measurements were made on each tissue sample and the arithmetic average was reported as the % additive content.

Tissue web samples 1 through 4 were made generally according to a wet press process. In order to adhere the tissue web to a creping surface, which in this embodiment comprised a Yankee dryer, additive compositions made according to the present disclosure were sprayed onto the dryer prior to contacting the dryer with the web. Two-ply or three-ply tissue products were produced. The samples were then subjected to various standardized tests and compared to commercial products.

Initially, softwood kraft (NSWK) pulp was dispersed in a pulper for 30 minutes at 400 consistency at about 37.8 °C (about 100 degrees F). Then, the NSWK pulp was transferred to a dump chest and subsequently diluted to approximately 3% consistency. Then, the NSWK pulp was refined at 3.36 kw-days (4.5 hp-days/metric ton). The above softwood fibers were utilized as the inner strength layer in a 3-layer tissue structure. The NSWK layer contributed approximately 34% of the final sheet weight.

Two kilograms KYMENE® 6500, available from Hercules, Incorporated, located in Wilmington, Delaware, U.S.A., per + ou (metric ton) of wood fiber was added to the furnish prior to the headbox.

Aracruz ECF, a eucalyptus hardwood Kraft (EHWK) pulp available from Aracruz, located in Rio de Janeiro, RJ, Brazil, was dispersed in a pulper for 30 minutes at about 4% consistency at about 37.8°C-(about 100 degrees Fahrenheit). The EHWK pulp was then transferred to a dump chest and subsequently diluted to about 3% consistency. The EHWK pulp fibers represent the two outer layers of the 3-layered tissue structure. The EHWK layers contributed approximately 66% of the final sheet weight.

Two kilograms KYMENE® 6500 per to a (metric ton)of wood fiber was added to the furnish prior to the headbox.

The pulp fibers from the machine chests were pumped to the headbox at a consistency of about 0.1 %. Pulp fibers from each machine chest were sent through separate manifolds in the headbox to create a 3-layered tissue structure. The fibers were deposited onto a felt in a Crescent Former.

The wet sheet, about 10-20% consistency, was adhered to a Yankee dryer, traveling at about 750 m (min) (about 2500 fpm) through a nip via a pressure roll. The consistency of the wet sheet after the pressure roll nip (post-pressure roll consistency or PPRC) was approximately 40%. The wet sheet adhered to the Yankee dryer due to the additive composition that is applied to the dryer surface. Spray booms situated underneath the Yankee dryer sprayed the additive composition, described in the present disclosure, onto the dryer surface at an addition level of 100 to 600 mg/m$^2$.

To prevent the felt from becoming contaminated by the additive composition, and to maintain desired sheet properties, a shield was positioned between the spray boom and the pressure roll.

The sheet was dried to about 95% - 98% consistency as it traveled on the Yankee dryer and to the creping blade. The creping blade subsequently scraped the tissue sheet and a portion of the additive composition off the Yankee dryer. The creped tissue base sheet was then wound onto a core traveling at about 600 m/min (about 1970 into soft rolls for converting. The resulting tissue base sheet had an air-dried basis weight of 14.2 g/m$^2$. Two or three soft rolls of the creped tissue were then rewound and plied together so that both creped sides were on the outside of the 2- or 3-ply structure. Mechanical crimping on the edges of the structure held the plies together. The plied sheet was then slit on the edges to a standard width of about 21.6 cm (approximately 8.5 inches) and folded. Tissue samples were conditioned and tested.

The additive composition that was applied to Sample Nos. 1 through 4 and tested is as follows:

| **Polymer** (wt. ratios in parentheses) | | | | **Dispersing Agent** | | | **Dispersing Agent conc. (wt.%)** |
|---|---|---|---|---|---|---|---|
| **AFFINITY™ EG8200/PRIMACOR™ 5986 (60/40)** | | | | **PRIMACOR™ 5986** | | | 40.0 |
| Polymer Particle size (μm) | Poly-dispersity | Solids (wt.%) | pH | Viscosity (cp) | Temp (oC) | RPM | Spindle |
| 0.71 | 2.12 | 40.0 | 11.3 | 448 | 22.1 | 50 | RV3 |

DOWICIL™ 75 antimicrobial, which is a preservative with the active composition of 96% cis 1-(3-chloroallyl)-3,5,7-triaza-1-azoniaadamantane chloride(also known as Quaternium-15) obtained from The Dow Chemical Company, was also present in each of the additive compositions.

The percent solids in solution for the different additive compositions was varied to deliver 100 to 600 mg/m$^2$ spray coverage on the Yankee Dryer. Varying the solids content in solution also varies the amount of solids incorporated into the base web. For instance, at 100 mg/m$^2$ spray coverage on the Yankee Dryer, it is estimated that about 1% additive composition solids is incorporated into the tissue web. At 200 mg/m$^2$ spray coverage on the Yankee Dryer, it is estimated that about 2% additive composition solids is incorporated into the tissue web. At 400 mg/m$^2$ spray coverage on the Yankee Dryer, it is estimated that about 4% additive composition solids is incorporated into the tissue web.

Prior to testing, all of the samples were conditioned according to TAPPI standards. In particular, the samples were placed in an atmosphere at 50% relative humidity and 22.2 °C (72°F) for at least four hours.

The following results were obtained:

| Sample No. | Identification of Control Samples | # plies | Basis Weight Bone Dry ($g/m^2$) | Basis Weight ($g/m^2$) | Additive Composition Coverage ($mg/m^2$) | GMT 9/7.62cm | GMT/Ply | HST (seconds) | xylene extraction add-on (%) | Stick-Slip Result |
|---|---|---|---|---|---|---|---|---|---|---|
| Control 1 | PUFF's Plus (Procter & Gamble) | 2 | | | 0 | | | | | -0 0**20** |
| Control 2 | CELEB Glycerin Treated Tissue (Nepia) | 2 | | | 0 | | | | | -0.019 |
| Control 3 | KLEENEX Ultra (Kimberly-Clark) | 3 | 39.21 | | 0 | 880 | 293 | 65.8 | | -0.018 |
| Control 4 | PUFFS(Procter& Gamble) | 2 | | | 0 | 672 | 336 | | | -0.018 |
| Control 5 | KLEENEX Lotion (Kimberly-Clark) | 3 | | | 0 | | | | | -0.017 |
| Control 6 | KLEENEX (Kimberly-Clark) | 2 | 26.53 | | 0 | 622 | 311 | 1.2 | | -0.012 |
| Control 7 | COTTONELLE Ultra (Kimberly-Clark) | 2 | | | 0 | | | | | -0.013 |
| Control 8 | ANDREX (Kimberly-Clark) | 2 | | | 0 | | | | | -0.017 |
| Control 9 | CHARMIN Ultra (Procter & Gamble) | 2 | | | 0 | | | | | -0,018 |
| Control 50 | CHARMIN Plus (Procter & Gamble) | 2 | | | 0 | | | | | -0.018 |
| Control 11 | CHARMIN Giant (Procter & Gamble) | 1 | | | 0 | | | | | -0.021 |
| 1 | | 2 | 27 32 | | 200 | 792 | 396 | 4 1 | 1.2 | 0.000 |

(continued)

| Sample No. | Identification of Control Samples | # plies | Basis Weight Bone Dry ($g/m^2$) | Basis Weight ($g/m^2$) | Additive Composition Coverage ($mg/m^2$) | GMT 9/7.62cm | GMT/Ply | HST (seconds) | xylene extraction add-on (%) | Stick-Slip Result |
|---|---|---|---|---|---|---|---|---|---|---|
| 2 | | 2 | 26.89 | | 400 | 775 | 388 | 7 | 41 | 0.016 |
| 3 | | 3 | 39.93 | | 400 | 1067 | 356 | 9.8 | 3.3 | 0.018 |
| 4 | | 2 | | | 431 | 874 | 437 | | 3.2* | 0.023 |

As shown above, the samples treated according to the present disclosure had good water absorbency rates as shown by the Hercules Size Test. In particular, samples made according to the present disclosure had an HST of well below 60 seconds, such as below 30 seconds, such as below 20 seconds, such as below 10 seconds. In fact, Sample No. 1 had an HST of less than about 5 seconds.
As also shown by the above table, samples made according to the present disclosure had superior Stick-Slip characteristics. As shown, samples made according to the present disclosure had a Stick-Slip of from about 0.000 to about 0.1. All of the comparative examples, on the other hand, had lower Stick-Slip numbers.

Example 2

In this example, base sheets made according to the present disclosure were tested in order to demonstrate the enhanced cleaning ability of the base sheets. In particular, the samples were subjected to a Cleaning Test which measures the amount of dirt particles, in milligrams, that a sample is capable of picking up.
More particularly, the Cleaning Test is described as follows.

Cleaning Test

The testing apparatus as shown in Fig. 9 and as explained in Example 1 above is used to conduct the Cleaning Test. In this test, however, the base sheet samples are clamped flat to the underside of the sled instead of a collagen film. Sifted potting soil is then placed upon the metal plate and the sled is slid across the metal plate in order to allow the base sheet sample to capture the potting soil.
More particularly, the procedure is as follows:

1) Base sheet samples (7.62 cm x 17.78 cm)-(3 inches by 7 inches)) are cut using a 7.62 cm (3") wide Precision cutter, weighed, and recorded in milligrams.
2) Samples are folded in half fastened to the 3.81 cm (1.5") sled with the treated side facing out on the bottom of the sled.
3) Sifted commercial potting soil is weighed out to 88 milligrams for each test. The potting soil comprises a mix of sphagnum peat moss, compost and perlite. One commercial source for the potting soil is "Premium Planters Mix" commercially available from Waupaca Materials. The potting soil was sifted using a suitable mesh sized screen that eliminated clumping and removed aggregates.
The sifted dirt is then positioned evenly on a 3.175 cm x 3.175 cm (1.25" x 1.25") template on the testing surface.
4) The sled with the base sheet sample is positioned 6.35 mm (¼ inch) behind the dirt at the same location each time and aligned with the pulley allowing for no slack.
5) A 100 gram weight (such as a brass weight) is positioned in the same spot for each test on top of the sled.
6) Test Works software is used. The tensile frame moves up and with the use or pulley, moves the sled over the test sample. The sled is moved 25.4 cm (10 inches) at a speed of 1.524 m/min (60 inches per minute).
7) After the test is done, the sample is carefully removed, weighed, and recorded. The amount of potting soil picked up by the sample in milligrams is determined. For each base sheet, two samples are made and tested and the results are averaged.

In this example, tissue samples were made similar to the samples described in Example 1 above. In particular, 2-ply creped samples were produced. The additive composition was applied to one side of the tissue samples in an amount of 13.3% by weight.
For purposes of comparison, a similar 2-ply tissue that did not contain the additive composition was tested. Instead of using the additive composition to crepe the tissue, a standard PVOH/KYMENE crepe package was used. The results are illustrated in Fig. 10.
As shown, the sample made in accordance with the present disclosure had a Cleaning Test Value of greater than 7 mg, while the Control had a Cleaning Test Value of 3 mg.

Example 3

The Cleaning Test described in Example 2 above was used to test hydroentangled webs. In particular, an untreated hydroentangled web was compared to a similar hydroentangled web that was treated with the additive composition.
In this example, the hydroentangled material used had a basis weight of 64 $g/m^2$ The hydroentangled base sheets contained 81% by weight cellulose fibers and 19% by weight spunbond fibers.
For the hydroentangled base sheets treated in accordance with the present disclosure, the additive composition

was applied to the base sheet generally using the process shown in Fig. 8. The additive composition used was the same that was used to treat the samples in Example 2 above. The additive composition was added to the base sheet in an amount of 1.51 g/$m^2$.

The treated hydroentangled base sheet was then subjected to the cleaning test described in Example 2 above along with a control sample that was untreated. The results are shown in Fig. 11. As shown, the control sample had a Cleaning Test Value of only 1 mg, while the sample made in accordance with the present disclosure had a Cleaning Test Value of greater than 8 mg. As shown, the presence of the additive composition increases the Cleaning Test Value for the base sheet in comparison to an identical untreated base sheet. Base sheets made in accordance with the present disclosure can have a Cleaning Test Value of greater than about 5 mg, such as greater than about 6 mg, such as greater than about 7 mg, such as even greater than about 8 mg.

## Claims

**1.** A sheet-like product comprising:

a base sheet having a first side and a second side, the base sheet comprising a coform web, having a bulk of greater than 3 cc/g and containing cellulose fibers and synthetic fibers, cellulose fibers being present in the base sheet in an amount from 70% to 90% by weight, 70% to 80% by weight being excluded; and
an additive composition present on at least the first side of the base sheet, the additive composition comprising a thermoplastic polymer comprising either an alpha-olefin interpolymer of ethylene and at least one comonomer or an alpha-olefin interpolymer of propylene and at least one comonomer, the additive composition further comprising a dispersing agent.

**2.** A sheet-like product as defined in claim 1, wherein the base sheet has a basis weight of less than 50 g/$m^2$.

**3.** A sheet-like product as defined in claim 1 or 2, wherein the additive composition has been applied to the first side of the base sheet and the base sheet has been creped after the additive composition has been applied.

**4.** A sheet-like product as defined in claim 1 or 2, wherein the additive composition has been first applied to a creping surface and then the first side of the base sheet is contacted with the creping surface in order to apply the additive composition to the first side.

**5.** A sheet-like product as defined in claim 3, wherein the additive composition has been applied to the first side of the base sheet according to a pattern prior to being creped.

**6.** A sheet-like product as defined in claim 1 or 2, wherein the product is substantially dry.

**7.** A sheet-like product as defined in claim 1 or 2, wherein the product comprises a wet wipe, the base sheet containing a cleaning solution.

**8.** A sheet-like product as defined in claim 1, further comprising a tissue web attached to the second side of the base sheet.

**9.** A sheet-like product as defined in any of the preceding claims, wherein the dispersing agent comprises an ethylene-carboxylic acid copolymer.

**10.** A sheet-like product as defined in claim 9, wherein the additive composition comprises the non-fibrous olefin polymer and wherein the olefin polymer comprises an alpha-olefin interpolymer of ethylene and at least one comonomer selected from the group consisting of a $C_4$ to $C_{20}$ linear, branched or cyclic diene, vinyl acetate, and a compound represented by the formula $H_2C{=}CHR$, wherein R is a $C_1$ to $C_{20}$ linear, branched or cyclic alkyl group or a $C_6$ to $C_{20}$ aryl group, or the alpha-olefin polymer comprises a copolymer of propylene with at least one comonomer selected from the group consisting of ethylene, a $C_4$ to $C_{20}$ linear, branched or cyclic diene, and a compound represented by the formula $H_2C{=}CHR$, wherein R is a $C_1$ to $C_{20}$ linear, branched or cyclic alkyl group or a $C_6$ to $C_{20}$ aryl group.

**11.** A sheet-like product as defined in claim 1 or 9, wherein the thermoplastic polymer comprises an interpolymer of ethylene and an alkene.

**12.** A sheet-like product as defined in any of the preceding claims, wherein the dispersing agent comprises a carboxylic acid, a salt of a carboxylic acid, a carboxylic acid ester, or a salt of a carboxylic acid ester.

## Patentansprüche

**1.** Blattförmiges Produkt, umfassend:

eine Basislage mit einer ersten Seite und einer zweiten Seite, wobei die Basislage eine Coformbahn umfasst, mit einem spezifischen Volumen von mehr als 3 $cm^3/g$, und enthaltend Cellulosefasern und Synthesefasern, wobei Cellulosefasern in der Basislage in einer Menge von 70 Gewichtsprozent bis 90 Gewichtsprozent, ausgenommen 70 Gewichtsprozent bis 80 Gewichtsprozent, vorhanden sind; und

eine auf der ersten Seite der Basislage vorhandene Additivzusammensetzung, wobei die Additivzusammensetzung ein thermoplastisches Polymer, umfassend entweder ein alpha-Olefin-Interpolymer von Ethylen und wenigstens einem Co-Monomer oder ein alpha-Olefin-Interpolymer von Propylen und wenigstens einem Co-Monomer, umfasst, wobei die Additivzusammensetzung des Weiteren ein Dispergiermittel umfasst.

**2.** Blattförmiges Produkt nach Anspruch 1, wobei die Basislage ein Flächengewicht von weniger als 50 $g/m^2$ hat.

**3.** Blattförmiges Produkt nach Anspruch 1 oder 2, wobei die Additivzusammensetzung auf die erste Seite der Basislage aufgetragen worden ist, und die Basislage gekreppt worden ist nachdem die Additivzusammensetzung aufgetragen worden ist.

**4.** Blattförmiges Produkt nach Anspruch 1 oder 2, wobei die Additivzusammensetzung zunächst auf eine Krepp-Oberfläche auftragen worden ist und die erste Seite der Basislage danach mit der Krepp-Oberfläche in Kontakt gebracht worden ist um die Additiv-Zusammensetzung auf die erste Seite aufzutragen.

**5.** Blattförmiges Produkt nach Anspruch 3, wobei vor dem Kreppen die Additivzusammensetzung in einem Muster auf die erste Seite der Basislage aufgetragen worden ist.

**6.** Blattförmiges Produkt nach Anspruch 1 oder 2, wobei das Produkt im Wesentlichen trocken ist.

**7.** Blattförmiges Produkt nach Anspruch 1 oder 2, wobei das Produkt ein Feuchttuch umfasst, wobei die Basislage eine Reinigungslösung enthält.

**8.** Blattförmiges Produkt nach Anspruch 1, des Weiteren umfassend eine an der zweiten Seite der Basislage befestigte Tissue-Bahn.

**9.** Blattförmiges Produkt nach einem der vorhergehenden Ansprüche, wobei das Dispergiermittel ein Ethylen-Carbonsäure-Copolymer umfasst.

**10.** Blattförmiges Produkt nach Anspruch 9, wobei die Additiv-Zusammensetzung das nicht-faserige Olefinpolymer umfasst, und wobei das Olefinpolymer ein alpha-Olefin-Interpolymer von Ethylen und wenigstens einem Co-Monomer umfasst, ausgewählt aus der Gruppe, bestehend aus einem linearen, verzweigten oder zyklischen $C_4$ bis $C_{20}$ Dien, Vinylacetat, und einer durch die Formel $H_2C{=}CHR$ dargestellten Verbindung, wobei R eine lineare, verzweigte oder zyklische $C_1$ bis $C_{20}$ Alkylgruppe oder eine $C_6$ bis $C_{20}$ Arylgruppe ist, oder wobei das alpha-Olefinpolymer ein Copolymer von Propylen mit wenigstens einem Co-Monomer umfasst, ausgewählt aus der Gruppe, bestehend aus Ethylen, einem linearen, verzweigten oder zyklischen $C_4$ bis $C_{20}$ Dien, und einer durch die Formel $H_2C{=}CHR$ dargestellten Verbindung, wobei R eine lineare, verzweigte oder zyklische $C_1$ bis $C_{20}$ Alkylgruppe oder eine $C_6$ bis $C_{20}$ Arylgruppe ist.

**11.** Blattförmiges Produkt nach Anspruch 1 oder 9, wobei das thermoplastische Polymer ein Interpolymer von Ethylen und einem Alken umfasst.

**12.** Blattförmiges Produkt nach einem der vorhergehenden Ansprüche, wobei das Dispergiermittel eine Carbonsäure, ein Salz einer Carbonsäure, einen Carbonsäureester, oder ein Salz eines Carbonsäureesters umfasst.

## Revendications

**1.** Produit analogue à une feuille comprenant :

une feuille de base ayant un premier côté et un second côté, la feuille de base comprenant un voile de coforme, ayant un volume massique supérieur à 3 $cm^3/g$ et contenant des fibres de cellulose et des fibres synthétiques, les fibres de cellulose étant présentes dans la feuille de base en une quantité de 70 % à 90 % en poids, 70 % à 80 % en poids étant exclus ; et

une composition d'additif présente sur au moins le premier côté de la feuille de base, la composition d'additif comprenant un polymère thermoplastique comprenant soit un interpolymère d'alpha-oléfine et d'éthylène et au moins un comonomère ou un interpolymère d'alpha-oléfine et de propylène et au moins un comonomère, la composition d'additif comprenant en outre un agent dispersant.

**2.** Produit analogue à une feuille tel que défini dans la revendication 1, dans lequel la feuille de base a une masse surfacique inférieure à 50 $g/m^2$.

**3.** Produit analogue à une feuille tel que défini dans la revendication 1 ou 2, dans lequel la composition d'additif a été appliquée sur le premier côté de la feuille de base et la feuille de base a été crêpée après que la composition d'additif a été appliquée.

**4.** Produit analogue à une feuille tel que défini dans la revendication 1 ou 2, dans lequel la composition d'additif a d'abord été appliquée à une surface de crêpage et ensuite le premier côté de la feuille de base est mis en contact avec la surface de crêpage afin d'appliquer la composition d'additif au premier côté.

**5.** Produit analogue à une feuille tel que défini dans la revendication 3, dans lequel la composition d'additif a été appliquée au premier côté de la feuille de base suivant un motif avant d'être crêpée.

**6.** Produit analogue à une feuille tel que défini dans la revendication 1 ou 2, dans lequel le produit est sensiblement sec.

**7.** Produit analogue à une feuille tel que défini dans la revendication 1 ou 2, dans lequel le produit comprend une lingette humide, la feuille de base contenant une solution de nettoyage.

**8.** Produit analogue à une feuille tel que défini dans la revendication 1, comprenant en outre un voile de papier mousseline attaché au second côté de la feuille de base.

**9.** Produit analogue à une feuille tel que défini dans l'une quelconque des revendications précédentes, dans lequel l'agent dispersant comprend un copolymère d'éthylène-acide carboxylique.

**10.** Produit analogue à une feuille tel que défini dans la revendication 9, dans lequel la composition d'additif comprend le polymère d'oléfine non fibreux et dans lequel le polymère d'oléfine comprend un interpolymère d'alpha-oléfine et d'éthylène et au moins un comonomère choisi parmi le groupe constitué d'un diène ramifié ou cyclique, linéaire en $C_4$ à $C_{20}$, d'un acétate vinylique et d'un composé représenté par la formule $H_2C$=CHR, dans laquelle R est un groupe alkyle ramifié ou cyclique, linéaire en $C_1$ à $C_{20}$, ou un groupe aryle en $C_6$ à $C_{20}$, ou le polymère d'alpha-oléfine comprend un copolymère de propylène avec au moins un comonomère choisi parmi le groupe constitué d'éthylène, d'un diène ramifié ou cyclique, linéaire en $C_4$ à $C_{20}$, et d'un composé représenté par la formule $H_2C$=CHR, dans laquelle R est un groupe alkyle ramifié ou cyclique, linéaire en $C_1$ à $C_{20}$, ou un groupe aryle en $C_6$ à $C_{20}$.

**11.** Produit analogue à une feuille tel que défini dans la revendication 1 ou 9, dans lequel le polymère thermoplastique comprend un interpolymère d'éthylène et d'un alcène.

**12.** Produit analogue à une feuille tel que défini dans l'une quelconque des revendications précédentes, dans lequel l'agent dispersant comprend un acide carboxylique, un sel d'un acide carboxylique, un ester d'acide carboxylique, ou un sel d'un ester d'acide carboxylique.

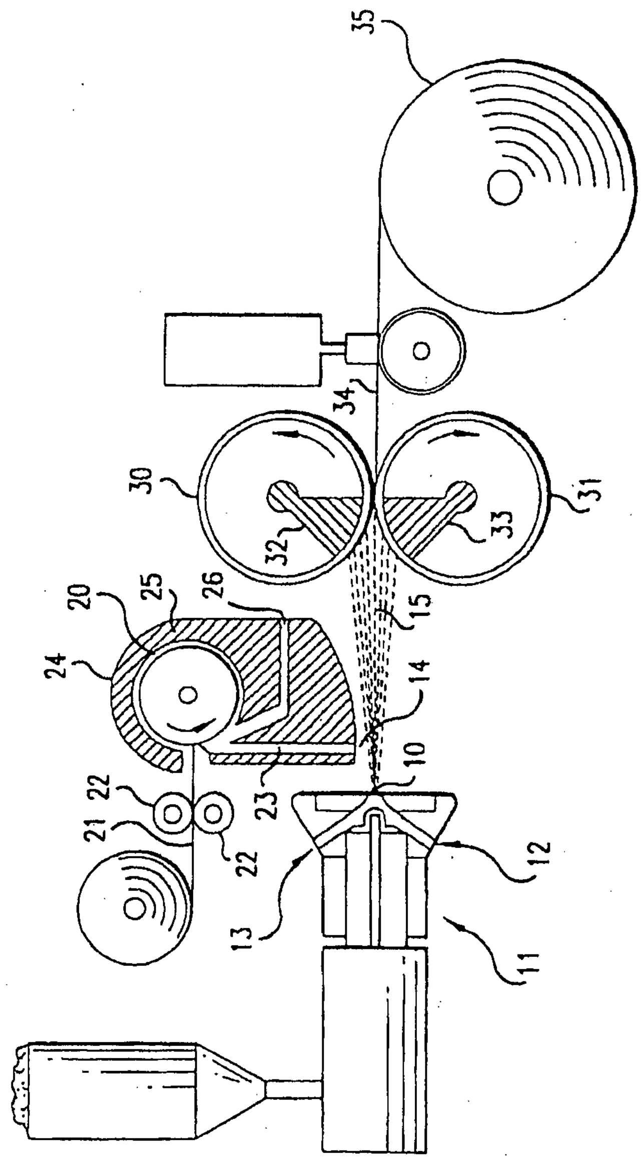


FIG. 1

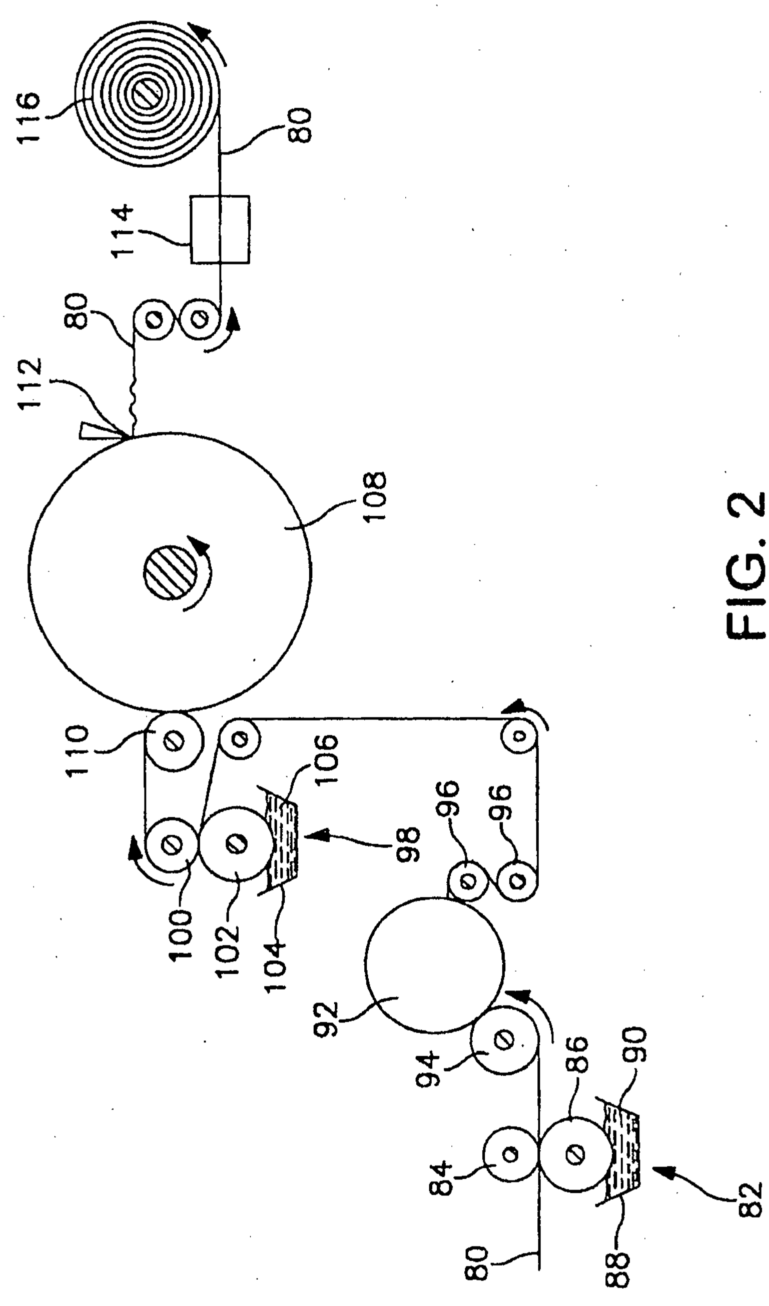
116
80
114
80
112
108
110
106
100
102
104
98
96
96
92
94
86
90
84
80
88
82

FIG. 2

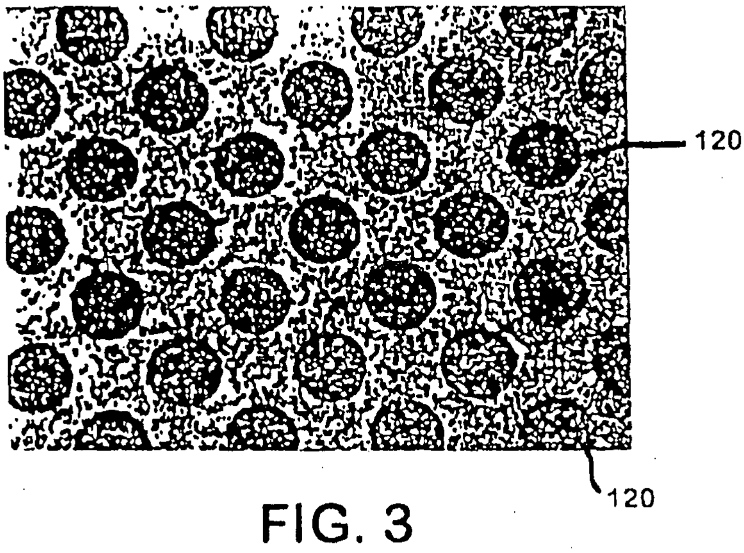


FIG. 3

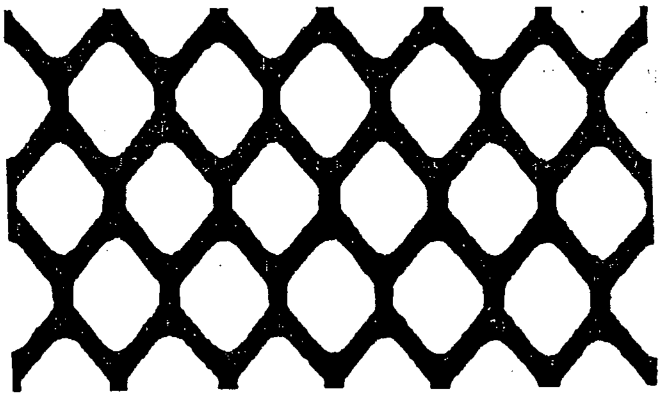

FIG. 4

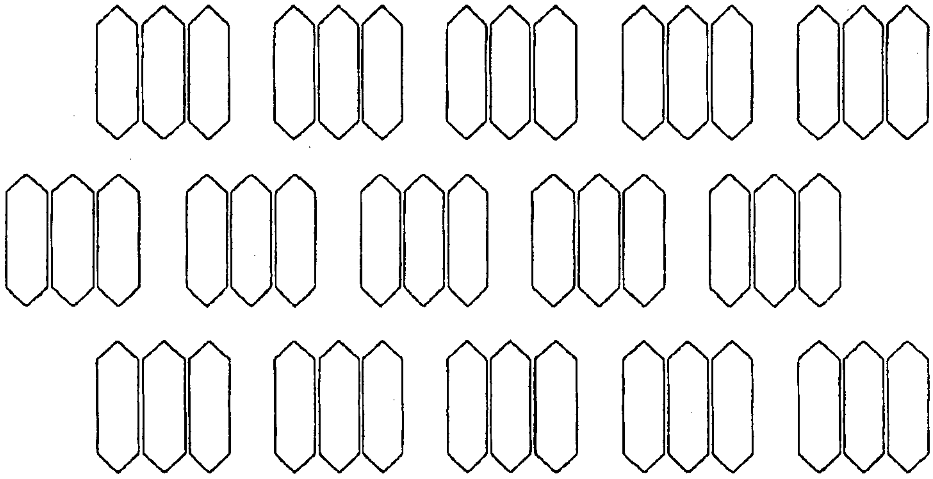

FIG. 5

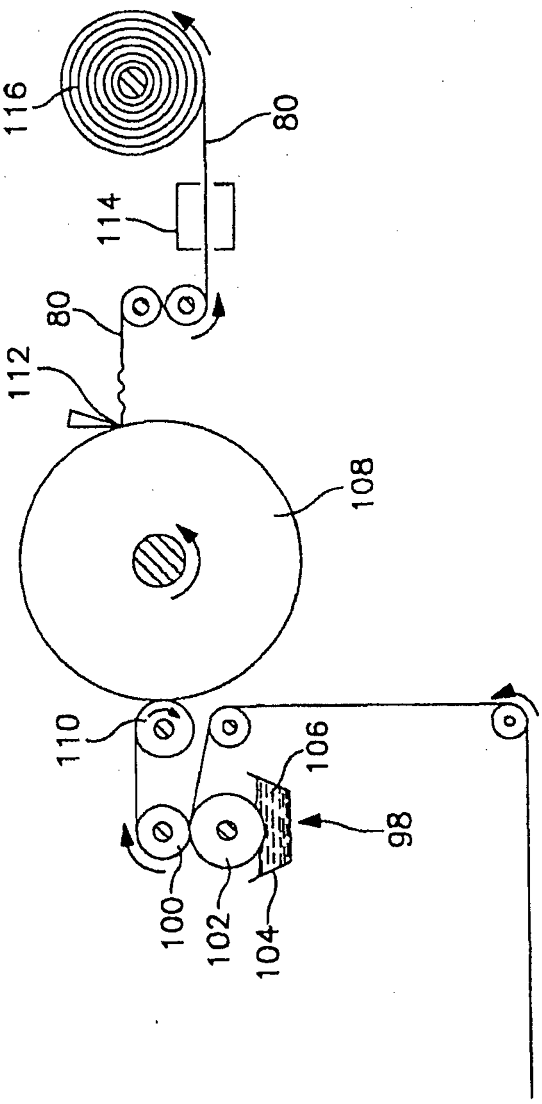


FIG. 6

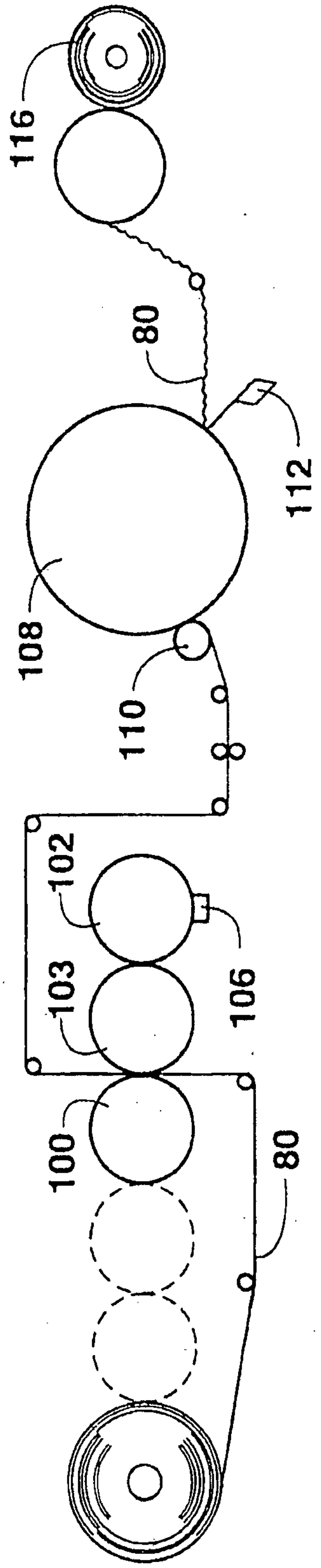


FIG. 7

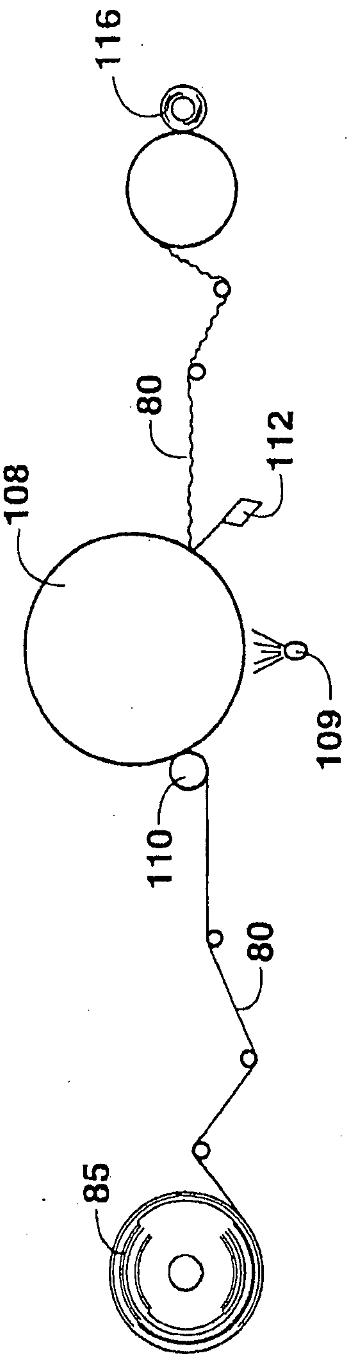


FIG. 8

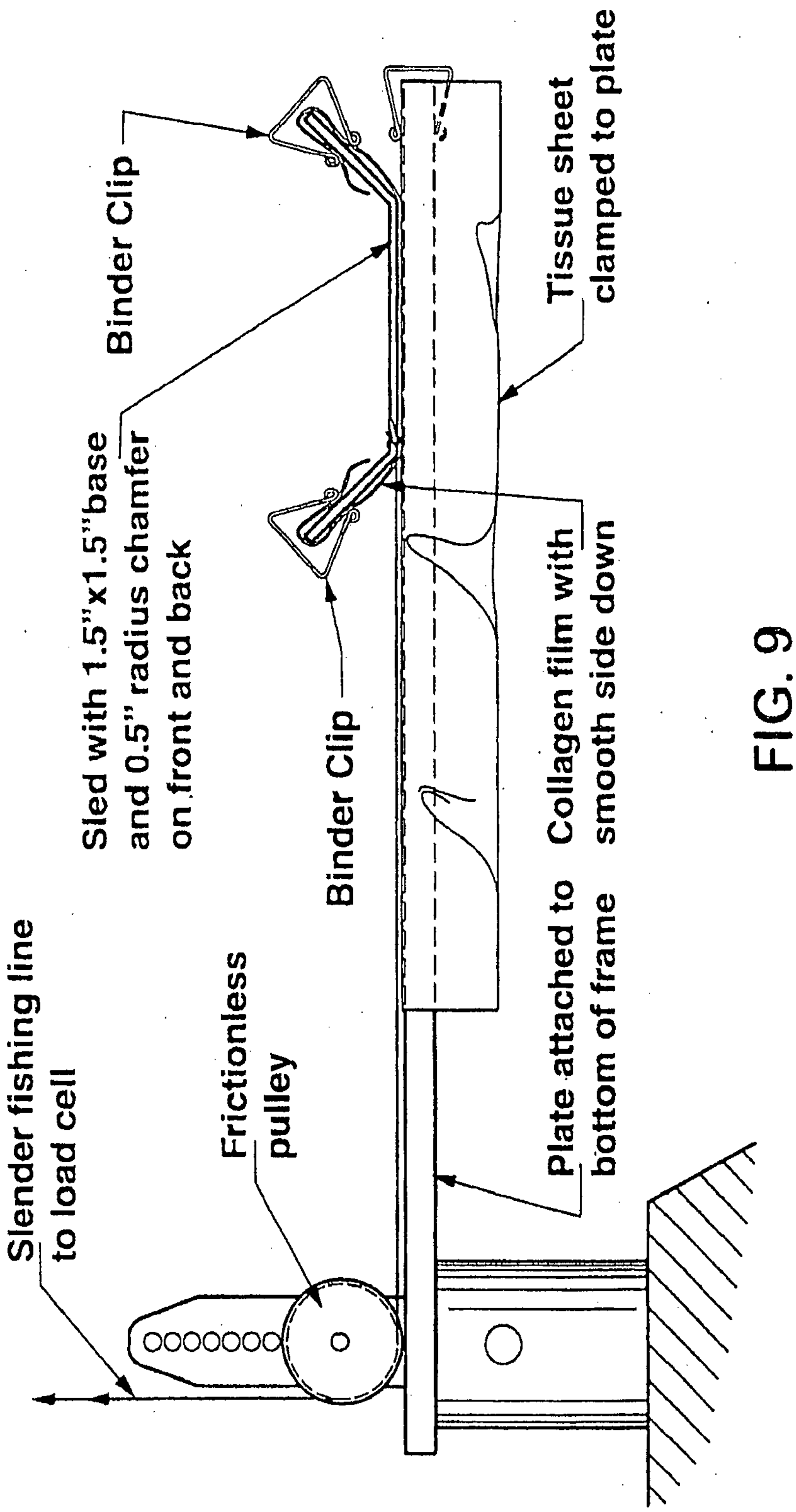
Binder Clip
Tissue sheet clamped to plate
Sled with 1.5"x1.5"base and 0.5" radius chamfer on front and back
Binder Clip
Collagen film with smooth side down
Slender fishing line to load cell
Frictionless pulley
Plate attached to bottom of frame

FIG. 9

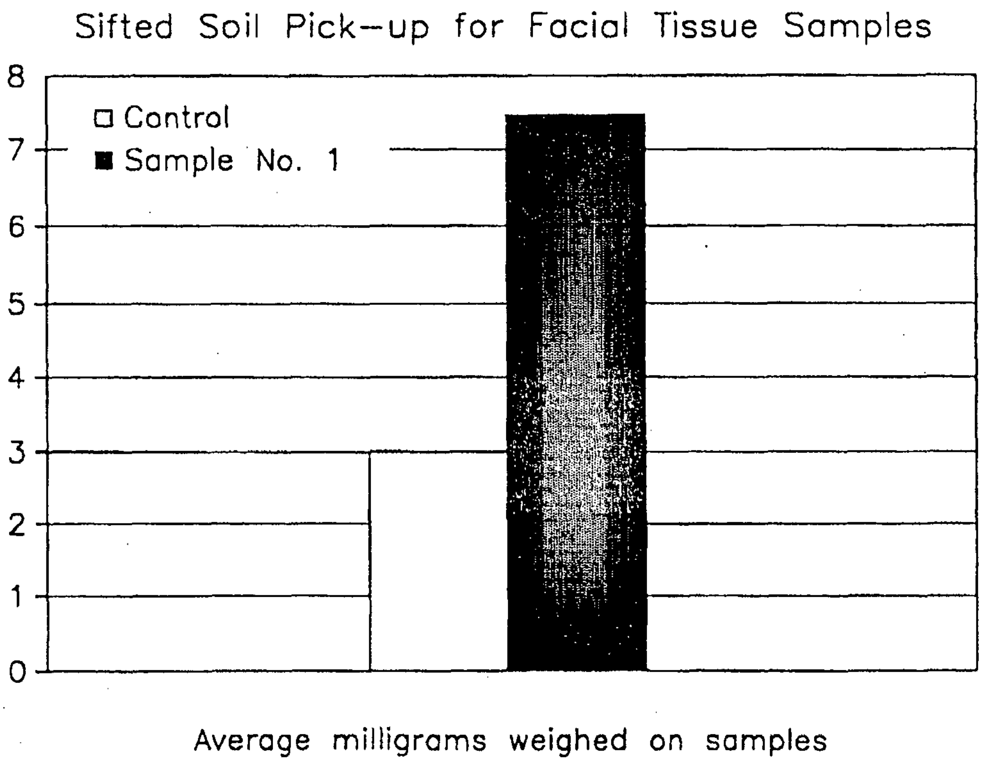
Sifted Soil Pick-up for Facial Tissue Samples
8
7
6
5
4
3
2
1
0
Control
Sample No. 1
Average milligrams weighed on samples

FIG. 10

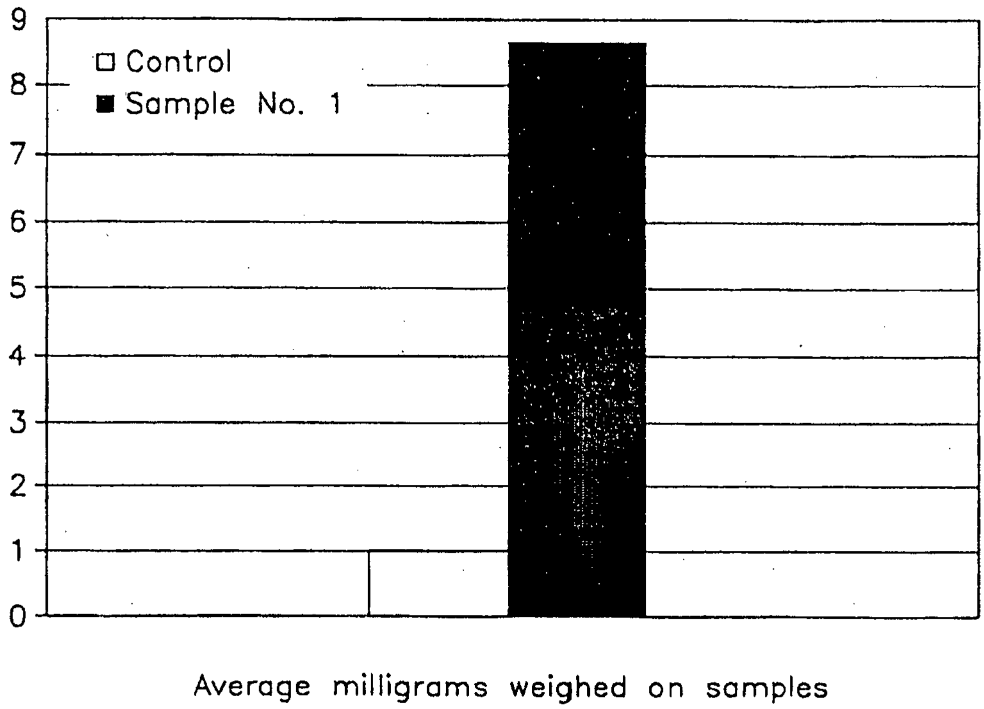
9
8
7
6
5
4
3
2
1
0
Control
Sample No. 1
Average milligrams weighed on samples

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4818464 A
- US 4100324 A
- US 20040099389 A1
- US 20040118544 A1
- WO 20070700145 A1
- WO 2007075356 A2
- WO 2008068653 A1
- WO 2008068659 A2
- WO 2008068652 A2
- WO 2008068652 A
- US 20050100754 A
- US 20050192365 A
- WO 2005021638 A
- WO 2005021622 A
- US 11303002 B
- US 11304490 B
- US 11303036 B
- US 11304998 B
- US 11304063 B
- US 11635385 B
- US 3645992 A, Elston
- US 4076698 A, Anderson
- US 5272236 A
- US 5278272 A
- US 4599392 A
- US 4988781 A
- US 5384373 A
- US 6538070 B
- US 6566446 B
- US 5869575 A
- US 6448341 B
- US 5677383 A
- US 6316549 B
- US 6111023 A
- US 5844045 A
- US 4514345 A, Johnson
- US 4528239 A, Trokhan
- US 5098522 A
- US 5260171 A, Smurkoski
- US 5275700 A, Trokhan
- US 5328565 A, Rasch
- US 5334289 A, Trokhan
- US 5431786 A, Rasch
- US 5496624 A, Steltjes, Jr.
- US 5500277 A, Trokhan
- US 5514523 A, Trokhan
- US 5554467 A, Trokhan
- US 5566724 A, Trokhan
- US 5624790 A, Trokhan
- US 5628876 A, Ayers
- US 5672248 A, Wendt
- US 5656132 A, Farrington
- US 6120642 A, Lindsay and Burazin
- US 6096169 A, Hermans
- US 6197154 B, Chen
- US 6143135 A, Hada